(19) Europäisches Patentamt
European Patent Office
Office européen des brevets

(11) EP 0 763 538 B1

(12) # EUROPÄISCHE PATENTSCHRIFT

(45) Veröffentlichungstag und Bekanntmachung des Hinweises auf die Patenterteilung:
**25.10.2000 Patentblatt 2000/43**

(21) Anmeldenummer: **96810582.5**

(22) Anmeldetag: **04.09.1996**

(51) Int Cl.[7]: **C07D 487/14**, C09B 57/00, C08K 5/3462
// (C07D487/14, 241:00, 209:00, 209:00)

(54) **Verfahren zum Färben von hochmolekularem Material mit polycyclischen Verbindungen**

Process for dyeing a high molecular material with polycyclic compounds

Procédé pour teindre un matériau de haut poids moléculaire avec des composés polycycliques

(84) Benannte Vertragsstaaten:
**CH DE FR GB LI**

(30) Priorität: **13.09.1995 CH 258595**
**22.09.1995 CH 267895**

(43) Veröffentlichungstag der Anmeldung:
**19.03.1997 Patentblatt 1997/12**

(73) Patentinhaber: **Ciba Specialty Chemicals Holding Inc.**
**4057 Basel (CH)**

(72) Erfinder:
- **Eichenberger, Thomas**
  **4056 Basel (CH)**
- **Ruch, Thomas**
  **1723 Marly (CH)**

(56) Entgegenhaltungen:
**EP-A- 0 035 358**
**EP-A- 0 163 609**
**DE-A- 2 144 568**
**FR-A- 2 650 590**

- **SEKIHACHI J ET AL: "SYNTHESIS AND CHROMOPHORIC PROPERTIES OF SYMMETRICAL BIS-HETEROANNELATED DIKETOPIPERAZINES: DIIMIDAZO- AND DIPYRAZOLO-PIPERAZINEDIONES" DYES AND PIGMENTS, Bd. 32, Nr. 1, 1.September 1996, Seiten 43-58, XP000620207**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 763 538 B1

## Beschreibung

Die Erfindung betrifft das Färben von Polymeren mit bestimmten polycyclischen Verbindungen, neue diese Verbindungen enthaltende Stoffzusammensetzungen sowie einzelne dieser polycyclischen Verbindungen selbst.

An Pigmente, die für einen breiten Einsatz in Polymeren geeignet sind, werden im allgemeinen folgende Anforderungen gestellt: hohe Reinheit des Farbtons (Sättigung), hohe Farbstärke und hohe Echtheiten, beispielsweise Licht- oder Hitzebeständigkeit. Die heute gebräuchlichen Gelbpigmente genügen zwar einzelnen dieser Anforderungen, weisen aber noch immer nicht alle erwünschten Eigenschaften auf.

Als polyzyklische Pigmente mit heterozyklischen Strukturen sind beispielsweise Colour Index Pigment Yellow 192, Pigment Orange 64 und Pigment Orange 67 bekannt. Dabei handelt es sich um einzelne hitzestabile Pigmente, deren koloristischen Eigenschaften wie Farbstärke und Sättigung zu wünschen übrig lassen. Ebenfalls bekannt sind 2,5-Diketopyrrolo-[3,4-c]-pyrrole wie Pigment Rot 254 (z.B. EP-A-163609), deren Farbe jedoch nur unter Verlust der pigmentären Eigenschaften nach Gelb verschoben werden kann.

Erst kürzlich berichteten J. Sekihachi und J. Griffiths über die Synthese und chromophorischen Eigenschaften symmetrischer Diketopiperazine, darunter einige erfindungsgemässe Verbindungen. Diese Publikation erschien am 1.9.1996 in Dyes and Pigments (Vol. 32/ N° 1 / S. 43-58).

In der Regel besitzen dabei nur solche Pigmente gute Eigenschaften, dessen Moleküle selbst eine hohe chemische Beständigkeit aufweisen und nur unter drastischen Bedingungen Reaktionen eingehen. Es wurde nun gefunden, dass bestimmte polycyclische Verbindungen, welche aufgrund ihrer chemischen Struktur leicht hydrolysierbar sind, überraschenderweise ausgezeichnete Eigenschaften als Farbmittel aufweisen.

Gegenstand der vorliegenden Erfindung ist nun ein Verfahren zum Färben von hochmolekularem organischem Material in der Masse, gekennzeichnet durch die Verwendung einer Verbindung der Formel (I)

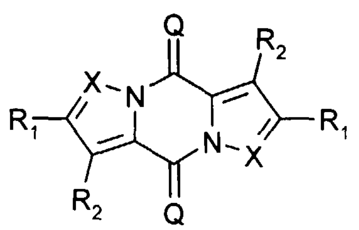

(I),

worin Q für O oder S, und X für $C(R_3)$ oder N stehen,
$R_1$, $R_2$ und $R_3$ unabhängig voneinander für Wasserstoff, Halogen, ausgewählt aus der Gruppe bestehend aus Chlor, Brom und Fluor, $C_1$-$C_6$Alkyl, $C_1$-$C_6$Alkoxy, Hydroxy, Cyan, Nitro, Amino, Morpholino, NH-$C_1$-$C_6$Alkyl,

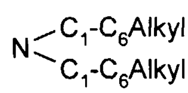

,

Halogen-$C_1$-$C_6$alkyl, NH-$E_1$, $A_1$ oder

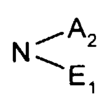

stehen,

worin $A_1$ und $A_2$ unabhängig voneinander mit 3 Resten $R_4$, $R_5$ und $R_6$ substituiertes $C_6$-$C_{12}$Aryl, oder mit 3 Resten $R_4$, $R_5$ und $R_6$ substituiertes $C_3$-$C_{12}$Heteroaryl, welches 1 bis 3 Heteroatome, ausgewählt aus der Gruppe bestehend aus N, O und S, im Ringsystem enthält, bedeuten,
und $E_1$ für $C_1$-$C_6$Alkyl,

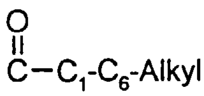 $A_1$, 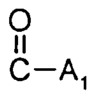 oder 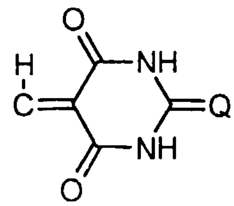

steht,
oder $R_1$ und $R_2$ zusammen oder $R_1$ und $R_3$ zusammen einen Rest $A_3$ darstellen, worin $A_3$ mit 3 Resten $R_7$, $R_8$ und $R_9$ substituiertes $C_4$-$C_8$Alkenylen oder $C_8$-$C_{12}$Cydoarylen bedeutet,
und $R_4$ bis $R_9$ unabhängig voneinander für Wasserstoff, Halogen, ausgewählt aus der Gruppe bestehend aus Chlor, Brom und Fluor, $C_1$-$C_6$Alkyl, $C_1$-$C_6$Alkoxy, Hydroxy, Cyan, Nitro, Amino, Morpholino, NH-$C_1$-$C_6$Alkyl,

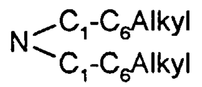 ,

Halogen-$C_1$-$C_6$alkyl,

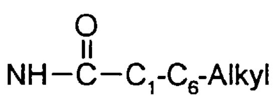 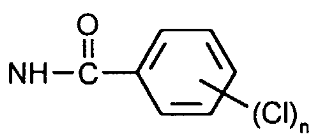 oder 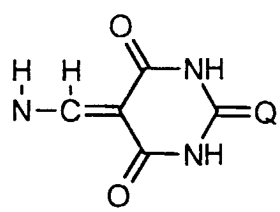

stehen,

worin n Null oder eine ganze Zahl von 1 bis 3 ist.

Formel (I) sowie die nachfolgend aufgeführten Formeln geben in einigen Fällen nur eine der möglichen tautomeren Strukturen wieder, auf welche sich die vorliegende Erfindung aber gegebenenfalls ebenfalls erstreckt. Die Verbindungen der Formel (I), beziehungsweise deren Tautomere, stellen Gelbpigmente dar, welche die oben erwähnten Anforderungen in hohem Masse erfüllen.

Bei $C_6$-$C_{12}$Aryl handelt es sich um isocyclische aromatische Reste, zum Beispiel Phenyl, 4-Biphenylyl, 1-Naphthyl oder 2-Naphthyl.

Bei $C_3$-$C_{12}$Heteroaryl handelt es sich um mehrfach ungesättigte heterocyclische Reste, zum Beispiel Pyridyl, Chinolyl, Isochinolyl, Bipyridyl, Triazinyl, Furyl, Benzofuryl, Dibenzofuryl, Pyrrolyl, Thienyl, Furfuryl, Isothiazolyl, Naphthyridinyl, Chinoxalinyl, Imidazolyl, Oxazolyl, Pyrazolyl, Pyrazinyl, Pyrimidinyl, Phenothiazinyl, Benzotriazolyl, Benzoxazolyl oder Carbazolyl, vorzugsweise um mono- und bicyclische heteroaromatische Reste.

Bei $C_4$-$C_8$Alkenylen handelt es sich um einen mehrfach ungesättigten linearen oder verzweigten Rest, zum Beispiel um 1,4-Buta-1,3-dienylen oder 2,5-Hexa-2,4-dienylen.

Bei $C_8$-$C_{12}$Arylen handelt es sich um mehrfach ungesättigte Reste mit aromatischem Charakter, welche mindestens einen Ring aufweisen, zum Beispiel um

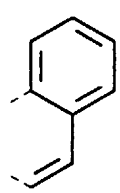 oder 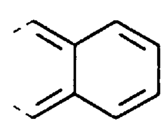 .

Halogen-$C_1$-$C_6$alkyl ist $C_1$-$C_6$Alkyl, dessen Wasserstoff- durch Halogenatome wie Brom, Chlor oder Fluor, partiell oder vollständig ersetzt sind. Als Beispiele für Halogen-$C_1$-$C_6$alkyl seien insbesondere Trichlormethyl, Fluor-

methyl, Trifluormethyl, Pentafluorethyl, β,β,β-Trifluorethyl, ω-Chlor-propyl, ω-Brom-butyl oder Perfluorhexyl genannt.

Folgende, beispielsweise aufgeführten Verbindungen der Formel (I) illustrieren die Erfindung in besonders anschaulicher Weise, ohne die Erfindung dabei in irgendeiner Art zu beschränken :

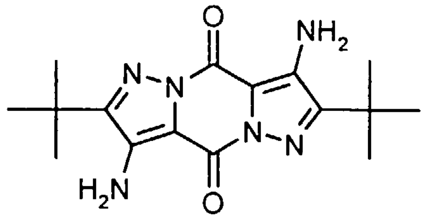

,

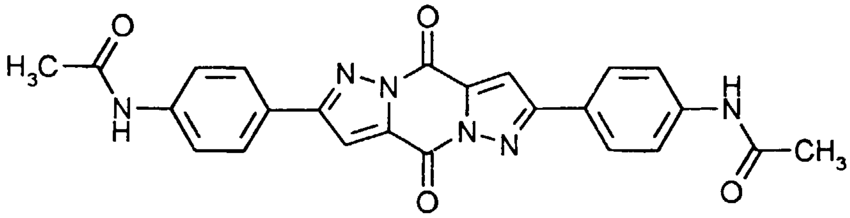

,

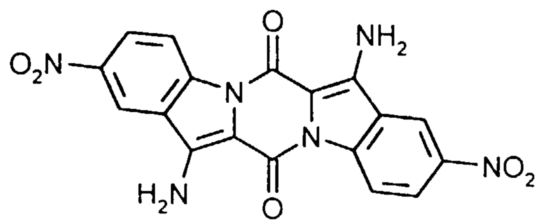

oder

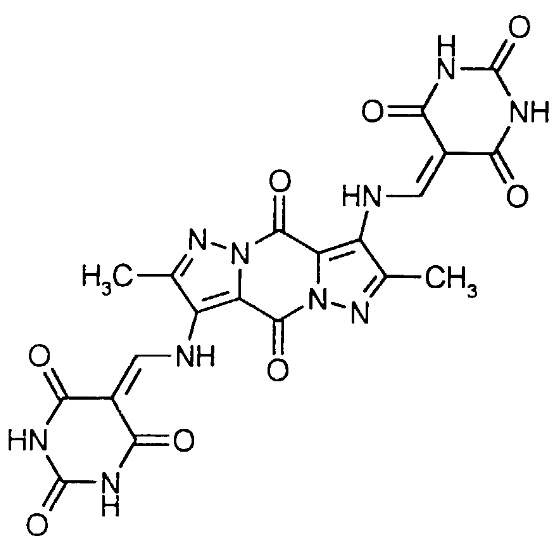

.

In

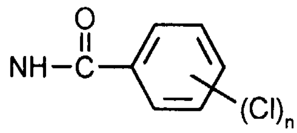

ist n bevorzugt Null oder eine ganze Zahl 1 oder 2, besonders bevorzugt Null, und die Chlorsubstituenten stehen gegebenenfalls insbesondere bevorzugt in o-, p- oder o,p-Stellung zur Amidgruppe.

Q steht bevorzugt für O.

Falls X für N steht, sind $R_1$ und $R_2$ bevorzugt $C_1$-$C_4$Alkyl, $C_1$-$C_4$Alkoxy, Amino, $A_4$, NH-$A_4$ oder NH-$E_1$, wobei $A_4$ einen Rest $A_1$, insbesondere bevorzugt mit 3 Resten $R_{10}$, $R_{11}$ und $R_{12}$ substituiertes $C_6$-$C_{12}$Aryl bedeutet, wobei $R_{10}$ bis $R_{12}$ unabhängig voneinander für $R_4$ bis $R_6$, insbesondere bevorzugt für Wasserstoff, $C_1$-$C_6$Alkoxy, Amino,

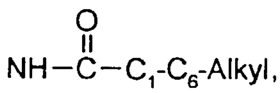, 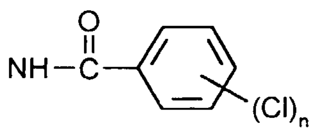 oder 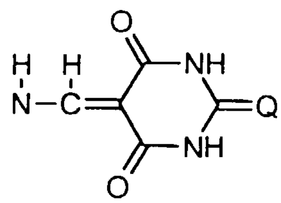

stehen.

Falls X für C($R_3$) steht, ist bevorzugt ein Rest $R_1$ oder $R_2$ $C_1$-$C_4$Alkyl, $C_1$-$C_4$Alkoxy, Amino, $A_4$, NH-$A_4$ oder NH-$E_1$, und $R_1$ bildet bevorzugt zusammen mit dem jeweils übrigen Rest $R_2$ oder insbesondere $R_3$ einen Rest

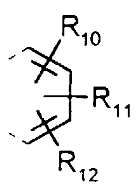, 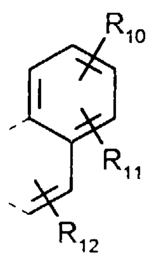 oder 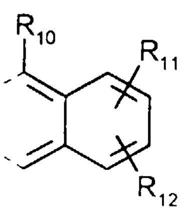,

wobei $A_4$, $E_1$ sowie $R_{10}$ bis $R_{12}$ die zuvor gegebene Definition haben.

Besonders bevorzugt ist die Verwendung der Verbindungen der Formel (I), worin

1] X für N steht, $R_1$ Methyl, Ethyl oder $A_4$ ist, und $R_2$ Amino oder NH-$E_1$ bedeutet, wobei $A_4$ und $E_1$ die zuvor gegebene Definition haben;

2] X für N steht, $R_1$ ein Rest $A_4$ ist, und $R_2$ Wasserstoff bedeutet, wobei $A_4$ die zuvor gegebene Definition hat;

3] X für N steht, $R_1$ Wasserstoff ist, und $R_2$ Amino, Morpholino, NH-$C_1$-$C_6$Alkyl ,

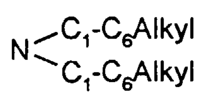, NH—$E_1$ oder 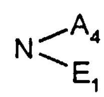

bedeutet, wobei $A_4$ und $E_1$ die zuvor gegebene Definition haben; oder

4] X für C($R_{13}$) steht, $R_1$ und $R_2$ zusammen einen Rest

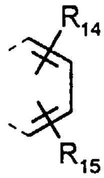

sind, $R_{13}$ Amino oder NH-$E_1$ bedeutet, und $R_{14}$ sowie $R_{15}$ unabhängig voneinander für Wasserstoff, Amino, NH-$E_1$ oder Nitro stehen, wobei $E_1$ die zuvor gegebene Definition hat.

Ganz besonders bevorzugt ist die Verwendung der Verbindungen der Formel (I), welche in mindestens einer tautomeren Form mindestens zwei je an einem Stickstoff gebundene Wasserstoffe aufweisen.

Die meisten Verbindungen der Formel (I) sind bekannt und können nach bekannten Methoden hergestellt werden. Beispielsweise kann man eine Verbindung der Formel (II)

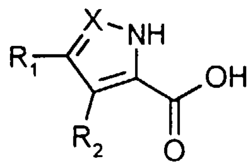 (II)

mit Dicyclohexylcarbodiimid und Pyridin unter Abspaltung von Wasser cyclodimerisieren, wie in DE-4102921 beschrieben, oder man kann eine Verbindung der Formel (III),

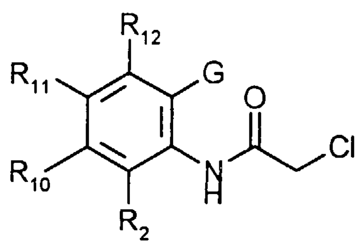 (III)

worin G beispielsweise für CN, CO-$C_1$-$C_6$Alkyl, CO-$A_1$ oder CO-O-$C_1$-$C_6$Alkyl steht,
nach der "Thorpe-Ziegler"-Methode zyklisieren, wie von H. Schäfer und K. Gewald in Journal für praktische Chemie 329/4, 745-748 (1987) beschrieben.

Noch unbekannte Verbindungen der Formel (I) können gegebenenfalls aus bekannten Substanzen nach bekannten Methoden hergestellt werden, oder in enger Analogie dazu. Man kann auch Verbindungen der Formel (I) in andere Verbindungen der Formel (I) nach bekannten Methoden überführen.

Die Wahl der geeignesten Methode richtet sich nach der Art der Substituenten.

Solche Änderungen der Funktionalität sind trivial und jedem Fachmann gut bekannt. Eine rasche Übersicht, welche Gruppen in welche anderen Gruppen umgewandelt werden können, vermittelt beispielsweise die Serie "Compendium of Organic Synthetic Methods" (John Wiley & Sons, ab 1971).

Bei solchen Veränderungen der Substituenten ohne Umwandlung des polycyclischen Gerüstes ist es bevorzugt, von einer zumindest teilweise löslichen Verbindung auszugehen, und sie in eine Verbindung der Formel (I) mit pigmentären Eigenschaften umzusetzen.

Die Gruppen Q können ebenfalls nachträglich verändert werden. Beispielsweise kann Oxo in Thioxo durch Behandlung mit Tetraphosphordecasulfid umgewandelt werden, womit Verbindungen der Formel (I), worin Q für S steht, erhalten werden können.

Unter den Verbindungen der Formel (I) sind auch solche, welche neu sind.

Gegenstand der vorliegenden Erfindung ist dementsprechend auch eine Verbindung der Formel (IV), (V) oder (VI),

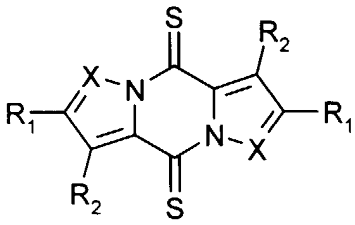 (IV) 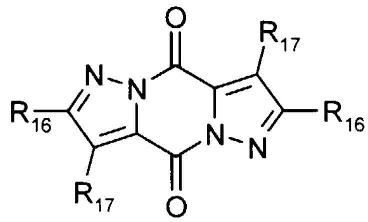 (V)

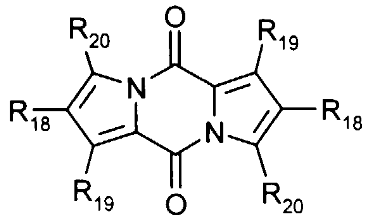 (VI)

worin in Formel (IV) X, $R_1$ und $R_2$ die gleiche Definition haben, wie in Formel (I).

Beansprucht sind folgende Alternativen von $R_{16}$ und $R_{17}$ in Formel (V), wo:

- ♦ $R_{16}$ $C_6$-$C_{12}$Aryl bedeutet, welches mindestens mit einem Rest $C_1$-$C_6$Alkoxy, Amino, Morpholino, NH-$C_1$-$C_6$Alkyl,

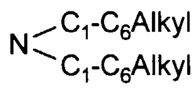 , NH—$E_2$ oder 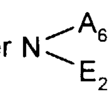

substituiert ist, und

$R_{17}$ unabhängig von $R_{16}$ für Wasserstoff, Chlor, Brom, Fluor, $C_1$-$C_6$Alkyl, $C_1$-$C_6$Alkoxy, Hydroxy, Cyan, Nitro, Amino, Morpholino,

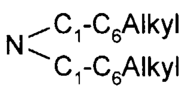 ,

Halogen-$C_1$-$C_6$alkyl, NH-$E_2$, $A_5$ oder

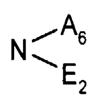

steht,

- • mit der Massgabe, dass $R_{17}$ nicht Wasserstoff ist, wenn $R_{16}$ eine Gruppe

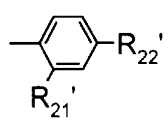

ist, worin $R_{21}$' für Alkoxy und $R_{22}$' für Wasserstoff, Alkyl, Alkoxy, Halogen oder Hydroxy, oder $R_{22}$' für Alkoxy und $R_{21}$' für Wasserstoff, Alkyl, Alkoxy, Halogen oder Hydroxy stehen;

oder

- ♦ $R_{16}$ Wasserstoff, Chlor, Brom, Fluor, $C_1$-$C_6$Alkyl, $C_1$-$C_6$Alkoxy, Hydroxy, Cyan, Nitro, Amino, Morpholino,

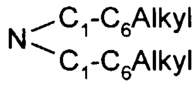, Halogen-$C_1$-$C_6$alkyl, NH—$E_2$, $A_5$ oder 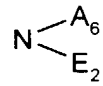

bedeutet, und
$R_{17}$ unabhängig von $R_{16}$ für $C_1$-$C_6$Alkoxy, Amino, Morpholino,

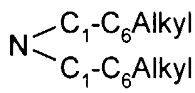,

NH-$E_2$,

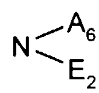

oder A5 steht;
oder

- ♦ $R_{16}$ und $R_{17}$ zusammen einen Rest $A_7$ darstellen,

wobei

$E_2$ für $C_1$-$C_6$Alkyl,

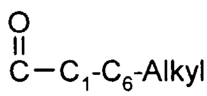, $A_5$, 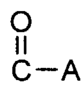 oder 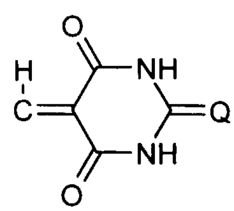

steht,

$A_5$ und $A_6$ unabhängig voneinander mit 3 Resten $R_{21}$, $R_{22}$ und $R_{23}$ substituiertes $C_6$-$C_{12}$Aryl, oder mit 3 Resten $R_{21}$, $R_{22}$ und $R_{23}$ substituiertes $C_3$-$C_{12}$Heteroaryl, welches 1 bis 3 Heteroatome, ausgewählt aus der Gruppe bestehend aus N, O und S, im Ringsystem enthält, bedeuten,

$A_7$ mit 3 Resten $R_{24}$, $R_{25}$ und $R_{27}$ substituiertes $C_4$-$C_8$Alkenylen oder $C_8$-$C_{12}$Aralkenylen bedeutet,

$R_{21}$ bis $R_{25}$ unabhängig voneinander für Wasserstoff, Chlor, Brom, Fluor, $C_1$-$C_6$Alkyl, $C_1$-$C_6$Alkoxy, Hydroxy, Cyan, Nitro, Amino, Morpholino, NH-$C_1$-$C_6$Alkyl,

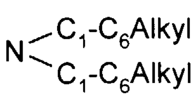, Halogen-$C_1$-$C_6$alkyl, 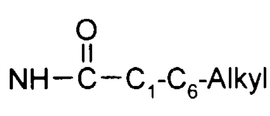, 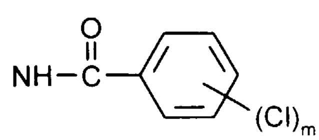

oder

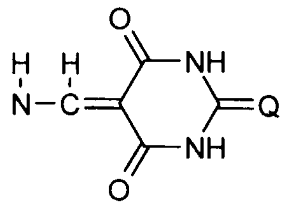

stehen, worin m Null oder eine ganze Zahl von 1 bis 3 ist;

$R_{27}$ für $C_1$-$C_6$Alkoxy steht, und

Q gleich O oder S ist.

Beansprucht sind folgende Alternativen von $R_{18}$, $R_{19}$ und $R_{20}$ in Formel (VI), wo:

- $R_{18}$ für Wasserstoff, Chlor, Brom, Fluor, $C_1$-$C_6$Alkyl, $C_1$-$C_6$Alkoxy, Hydroxy, Cyan, Nitro, Amino, Morpholino,

  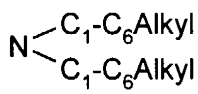 , Halogen-$C_1$-$C_6$alkyl, NH—$E_3$, $A_8$ oder 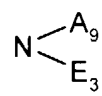,

  $R_{19}$ für $C_1$-$C_6$Alkoxy, Amino, Morpholino, NH-$C_1$-$C_6$Alkyl,

  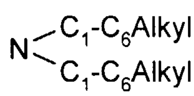 ,

  NH-$E_3$, $A_8$
  oder

  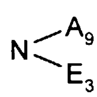,

  und
  $R_{20}$ für Wasserstoff, Chlor, Brom, Fluor, $C_1$-$C_6$Alkyl, $C_1$-$C_6$Alkoxy, Hydroxy, Cyan, Nitro, Amino, Morpholino,

  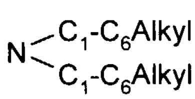 , Halogen-$C_1$-$C_6$alkyl, NH—$E_3$ oder 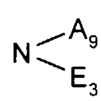

  stehen;
  oder

- $R_{18}$ für $C_1$-$C_6$Alkoxy, Amino, Morpholino, NH-$C_1$-$C_6$Alkyl,

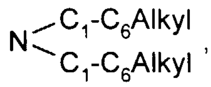

NH-$E_3$ oder

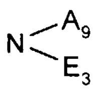

steht,

$R_{19}$ für Wasserstoff, Chlor, Brom, Fluor, $C_1$-$C_6$Alkyl, $C_1$-$C_6$Alkoxy, Hydroxy, Cyan, Nitro, Amino, Morpholino,

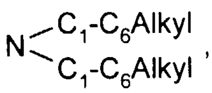 , Halogen-$C_1$-$C_6$alkyl, NH—$E_3$, $A_8$ oder 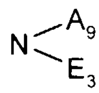

steht, und

$R_{20}$ ein Rest $A_8$ ist;

oder

- $R_{18}$ und $R_{19}$ unabhängig voneinander für Wasserstoff, Chlor, Brom, Fluor, $C_1$-$C_6$Alkyl, $C_1$-$C_6$Alkoxy, Hydroxy, Cyan, Nitro, Amino, Morpholino

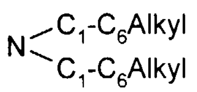

Halogen-$C_1$-$C_6$alkyl, NH-$E_3$, $A_8$ oder

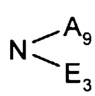

stehen, und

$R_{20}$ ein Rest $A_{12}$ ist;

oder

- $R_{18}$ und $R_{19}$ zusammen einen Rest $A_{11}$ darstellen, und

$R_{20}$ für Wasserstoff, Halogen, Chlor, Brom, Fluor, $C_1$-$C_6$Alkyl, $C_1$-$C_6$Alkoxy, Hydroxy, Cyan, Nitro, Amino, Morpholino,

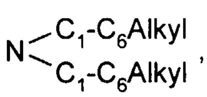

Halogen-$C_1$-$C_6$alkyl, NH-$E_3$, $A_8$ oder

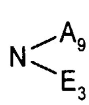

steht,
oder

- ♦ $R_{18}$ und $R_{20}$ zusammen einen Rest $A_{11}$ darstellen, und
  $R_{19}$ für $C_1$-$C_6$Alkyl oder Amino steht,
  oder

- ♦ $R_{18}$ und $R_{20}$ zusammen einen Rest $A_{10}$ darstellen, und
  $R_{19}$ für Chlor, Brom, Fluor, $C_1$-$C_6$Alkoxy, Hydroxy, Cyan, Nitro, Morpholino,

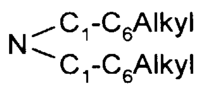 , Halogen-$C_1$-$C_6$alkyl, NH—$E_3$, $A_8$ oder 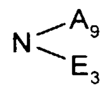

steht,

- mit der Massgabe, dass es sich bei der Verbindung der Formel (VI) nicht um eine Verbindung der Formel

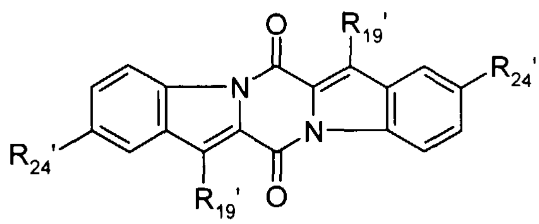

(XXIV) handelt, worin
wenn $R_{24}$' gleich Wasserstoff, $R_{19}$' gleich Cyano oder Hydroxy, oder wenn $R_{24}$' gleich Chlor, $R_{19}$' gleich Phenyl, o-Chlor-phenyl oder o-Fluor-phenyl sind;

worin

$E_3$ für $C_1$-$C_6$Alkyl,

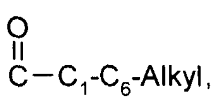, $A_8$, 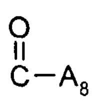 oder 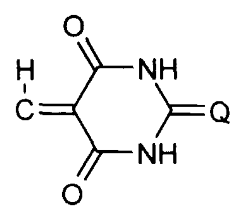

steht;

$A_8$ und $A_9$ unabhängig voneinander mit 3 Resten $R_{21}$, $R_{22}$ und $R_{23}$ substituiertes $C_6$-$C_{12}$Aryl, oder mit 3 Resten $R_{21}$, $R_{22}$ und $R_{23}$ substituiertes $C_3$-$C_{12}$Heteroaryl, welches 1 bis 3 Heteroatome, ausgewählt aus der Gruppe bestehend aus N, O und S, im Ringsystem enthält, bedeuten;

$A_{10}$ mit 3 Resten $R_{24}$, $R_{25}$ und $R_{26}$ substituiertes $C_4$-$C_8$Alkenylen oder $C_8$-$C_{12}$Arylen bedeutet;

$A_{11}$ mit 3 Resten $R_{24}$, $R_{25}$ und $R_{27}$ substituiertes $C_4$-$C_8$Alkenylen oder $C_8$-$C_{12}$Arylen bedeutet,

$A_{12}$ mit 3 Resten $R_{21}$, $R_{22}$ und $R_{28}$ substituiertes $C_6$-$C_{12}$Aryl, oder mit 3 Resten $R_{21}$, $R_{22}$ und $R_{28}$ substituiertes $C_3$-$C_{12}$Heteroaryl, welches 1 bis 3 Heteroatome, ausgewählt aus der Gruppe bestehend aus N, O und S, im Ringsystem enthält, bedeutet;

$R_{21}$ bis $R_{26}$ unabhängig voneinander für Wasserstoff, Chlor, Brom, Fluor, $C_1$-$C_6$Alkyl, $C_1$-$C_6$Alkoxy, Hydroxy, Cyan, Nitro, Amino, Morpholino, NH-$C_1$-$C_6$Alkyl,

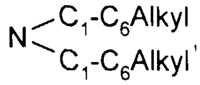

Halogen-$C_1$-$C_6$alkyl,

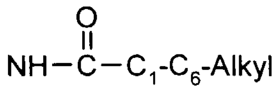, 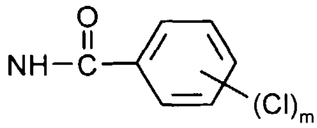 oder 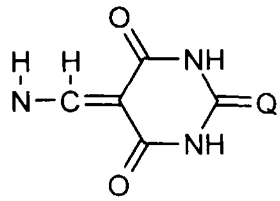

stehen;

$R_{27}$ und $R_{28}$ unabhängig voneinander für $C_1$-$C_6$Alkoxy, Amino, Morpholino, NH-$C_1$-$C_6$Alkyl,

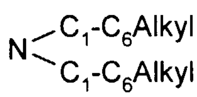, NH—$E_3$ oder 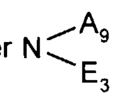

stehen;

m Null oder eine ganze Zahl von 1 bis 3 ist; und

Q für O oder S steht.

Die Verbindungen der Formeln (IV), (V) und (VI) eignen sich als Farbmittel. Bevorzugt sind solche Verbindungen der Formeln (IV), (V) und (VI), welche mindestens eine Gruppe $C_1$-$C_6$Alkoxy, Amino, Morpholino, NH-$C_1$-$C_6$Alkyl,

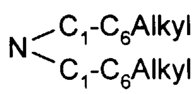, NH—$E_1$ oder 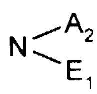

enthalten, wobei $A_1$, $A_2$ und $E_1$ die bei Formel (I) gegebene Definition besitzen.

Je nach Art ihrer Substituenten und des zu färbenden Polymeren können die Verbindungen der Formeln (I), (IV), (V) und (VI) als polymerlösliche Farbstoffe oder insbesondere als Pigmente verwendet werden. Im letzteren Falle ist es vorteilhaft, die bei der Synthese anfallenden Produkte in eine feindisperse Form überzuführen. Dies kann auf verschiedene Weise geschehen, beispielsweise :

a] durch Mahlen oder Kneten, zweckmässig in Gegenwart von Mahlhilfsmitteln, wie anorganischen oder organischen Salzen mit oder ohne Zusatz organischer Lösungsmittel; nach dem Mahlen werden die Hilfsmittel wie üblich entfernt, beispielsweise lösliche anorganische Salze durch Waschen mit Wasser und wasserunlösliche organische Lösungsmittel durch Wasserdampfdestillation,

b] durch Umfällen aus Schwefelsäure, Methansulfonsäure, Trichloressigsäure oder Polyphosphorsäure.

Es kann sich als zweckmässig erweisen, die rohen Pigmente oder die nach a] oder b] behandelten Pigmente mit organischen Lösungsmitteln, vorzugsweise mit solchen, die über 100°C sieden, nachzubehandeln. Diese Behandlung dauert üblicherweise von ¼ bis 72 Stunden und kann gegebenenfalls bei erhöhter Temperatur, beispielsweise bei

30 bis 200°C, erfolgen.

Als besonders geeignet erweisen sich, durch Halogenatome, Alkyl- oder Nitrogruppen substituierte Benzole, wie Xylole, Chlorbenzol, o-Dichlorbenzol oder Nitrobenzol sowie Pyridinbasen, wie Pyridin, Picolin oder Chinolin, ferner Ketone, wie Cyclohexanon, Äther wie Ethylenglykolmonomethyl- oder -monoethyläther, Amide, wie Dimethylformamid oder N-Methyl-pyrrolidon, sowie Dimethylsulfoxyd, Sulfolan oder Wasser allein, gegebenenfalls unter Druck. Man kann die Nachbehandlung auch in Wasser in Gegenwart von organischen Lösungsmitteln und/oder mit Zusatz von oberflächenaktiven Substanzen durchführen.

Die Pigmente weisen vorzugsweise eine BET-Oberfläche [bestimmt nach DIN 66132, *Bestimmung der spezifischen Oberfläche von Feststoffen durch Stickstoffadsorption / 1.- Differenzverfahren nach Haul und Dümbgen* (Ausgabe Juli 1975)] von 5-150 $m^2$/g auf. Pigmente, die eine BET-Oberfläche von 5-30 $m^2$/g aufweisen, haben eher dekkenden Charakter, während solche mit BET-Oberflächen von 30-150 $m^2$/g eher transparent sind. Die BET-Oberfläche und die Korngrössenverteilung lässt sich durch die oben erwähnten Nachbehandlungen steuern.

Je nach Verwendungszweck erweist es sich als vorteilhaft, die Pigmente als Toner oder in Form von Präparaten zu verwenden. Je nach Konditionierverfahren oder Applikationszweck kann es von Vorteil sein, dem Pigment gewisse Mengen an texturverbessemden Mitteln vor oder nach dem Konditionierprozess zuzufügen, sofern diese keine negative Wirkung bei der Verwendung der Pigmente zur Färbung von hochmolekularen organischen Materialen, insbesondere Polyethylen, haben. Als solche kommen insbesondere Fettsäuren mit mindestens 18 C-Atomen, beispielsweise Stearin- oder Behensäure, oder deren Amide oder Metallsalze, insbesondere Mg-Salze, sowie Weichmacher, Wachse, Harzsäuren, wie Abietinsäure, Kolophoniumseife, Alkylphenole oder aliphatische Alkohole, wie Stearylalkohol oder aliphatische 1,2-Dihydroxy-verbindungen mit 8 bis 22 C-Atomen, wie 1,2-Dodecandiol, ferner modifizierte Kolophoniummaleinatharze oder Fumarsäurekolophoniumharze in Betracht. Die texturverbessernden Mittel werden vorzugsweise in Mengen von 0,1 bis 30 Gew.-%, insbesondere 2 bis 15 Gew.-%, bezogen auf das Endprodukt, zugesetzt. Die obenerwähnten 1,2-Dihydroxy-verbindungen, insbesondere 1,2-Dodecandiol, können auch zur Verbesserung der Filtration der suspendierten Pigmentzusammensetzung eingesetzt werden.

Bezogen auf das zu färbende hochmolekulare organische Material kann die Verbindung der Formel (I) in einer Menge von 0,01 bis 30 Gew.-%, vorzugsweise 0,1 bis 10 Gew.-%, eingesetzt werden.

Einen weiteren Erfindungsgegenstand bilden deshalb Stoffzusammensetzungen, enthaltend eine Verbindung der Formel (I) und ein hochmolekulares organisches Material.

Von besonderem Interesse oder bevorzugt sind unter diesen Stoffzusammensetzungen jene, die eine Verbindung der Formel (I) enthalten, welche bei den oben als von besonderem Interesse oder bevorzugt genannten Verfahren zum Färben von hochmolekularem organischen Material verwendet wird.

Zur Färbung von organischen Materialen können die Verbindungen der Formel (I) einzeln gebraucht werden. Es ist aber ebenfalls möglich, mehrere Verbindungen der Formel (I) gleichzeitig als Gemische einzusetzen, oder sogenannte feste Lösungen oder Mischkristalle zu gebrauchen, worin eine Verbindung der Formel (I) und eine andere Verbindung der Formel (I), oder eine Verbindung der Formel (I) und ein Farbmittel einer anderen chemischen Klasse, zusammen eingebettet sind.

Das erfindungsgemäss zu färbende hochmolekulare organische Material kann natürlicher oder künstlicher Herkunft sein. Es kann sich z.B. um Naturharze oder trocknende Öle, Gummi oder Casein oder um abgewandelte Naturstoffe, wie Chlorkautschuk, ölmodifizierte Alkydharze, Viscose, um Celluloseäther oder -Ester, wie Ethylcellulose, Celluloseacetat, Cellulosepropionat, Celluloseacetobutyrat oder Nitrocellulose handeln, insbesondere aber um vollsynthetische organische Polymere (Duroplaste und Thermoplaste), wie sie durch Polymerisation, Polykondensation oder Polyaddition erhalten werden. Aus der Klasse der Polymerisationsharze seien in erster Linie genannt, Polyolefine, wie Polyethylen, Polypropylen oder Polyisobutylen, ferner substituierte Polyolefine, wie Polymerisate aus Vinylchlorid, Vinylacetat, Styrol, Acrylnitril oder Acrylsäure- und/oder Methacrylsäureester oder Butadien, sowie Kopolymerisate der erwähnten Monomeren, insbesondere ABS oder EVA.

Aus der Reihe der Polyadditionsharze und Polykondensationsharze seien die Kondensationsprodukte von Formaldehyd mit Phenolen, die sogenannten Phenoplaste, und die Kondensationsprodukte von Formaldehyd mit Harnstoff, Thioharnstoff und Melamin, die sogenannten Aminoplaste, die als Lackharze verwendeten Polyester, und zwar sowohl gesättigte, wie z.B. Alkydharze, als auch ungesättigte, wie beispielsweise Maleinatharze, ferner die linearen Polyester und Polyamide, Polyurethane oder Silikone genannt.

Die erwähnten hochmolekularen Verbindungen können einzeln oder in Gemischen, als plastische Massen oder Schmelzen, die gegebenenfalls zu Fasem versponnen werden können, vorliegen.

Sie können auch in Form ihrer Monomeren oder im polymerisierten Zustand in gelöster Form als Filmbildner oder Bindemittel für Lacke oder Druckfarben vorliegen, wie z.B. Leinölfirnis, Nitrocellulose, Alkydharze, Melaminharze und Harnstoff-Formaldehydharze oder Acrylharze.

Die Pigmentierung der hochmolekularen, organischen Substanzen mit den Pigmenten der Formel (I) erfolgt beispielsweise derart, dass man ein solches Pigment gegebenenfalls in Form von Masterbatches, diesen Substraten unter Verwendung von Walzwerken, Misch- oder Mahlapparaten zumischt. Das pigmentierte Material wird hierauf nach

an sich bekannten Verfahren wie Kalandrieren, Pressen, Strangpressen, Streichen, Giessen oder durch Spritzguss in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können im erfindungsgemässen Verfahren vor oder nach der Einverleibung des Pigmentfarbstoffes in die Polymeren eingearbeitet werden. Es ist ferner möglich, zwecks Erzielung verschiedener Farbtöne den hochmolekularen, organischen Stoffen neben den Verbindungen der Formel (I) noch Füllstoffe bzw. andere farbgebende Bestandteile wie Weiss-, Bunt- oder Schwarzpigmente in beliebigen Mengen zuzufügen.

Zum Pigmentieren von Lacken und Druckfarben werden die hochmolekularen organischen Materialien und die Verbindungen der Formel (I) gegebenenfalls zusammen mit Zusatzstoffen wie Füllmitteln, anderen Pigmenten, Siccativen oder Weichmachern, in einem gemeinsam organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken oder Drucken, zeichnen sich durch einen gelben bis orangeroten Farbton, eine sehr grosse Farbstärke, hohe Sättigung, gute Dispergierbarkeit, gute Überlackier-, Migrations-, Hitze-, Licht- und Wetterechtheit sowie durch einen guten Glanz und gutes IR-Remissionsverhalten aus. Besonders interessant sind die erfindungsgemässen Gelbpigmente infolge ihres neutralgelben Farbtons in Verbindung mit einer hohen Farbstärke, Sättigung und hohen Echtheiten.

Handelt es sich beim zu färbenden hochmolekularen Material um einen Lack, so kann es sich um eine gewöhnlichen Lack, oder auch um einen Speziallack, beispielsweise einen Automobillack, bevorzugt um eine beispielsweise Metall- oder Mica-Partikel enthaltende Metalleffektlackierung, handeln.

Die Verbindungen der Formel (I) können auch als photoelektrophoretische Toner verwendet werden.

Wenn die Verbindungen der Formel (I) in den angewandten Polymeren gelöst vorliegen, zeichnen sie sich ebenfalls durch reinen Farbton, grosse Farbstärke, hohe Lichtechtheit und ausserdem durch hohe Fluoreszenz aus. Sie eignen sich in Sonnenenergie-Kollektoren und zur Herstellung von Laser-Strahlen.

Die nachfolgenden Beispiele verdeutlichen die Erfindung:

Beispiel 1: 11,8 g der Verbindung der Formel (VII)

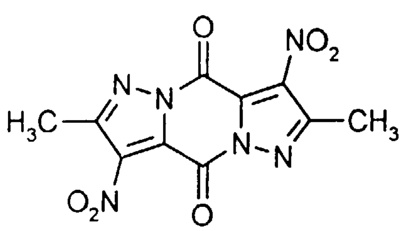

(VII),

(hergestellt nach Beilstein E III/IV 26, 2508) werden in 120 ml Dimethylformamid (DMF) aufgeschlämmt und unter Zusatz von 1,2 g Palladium/Aktivkohle (10 Gew.-%) bei 50°C unter Normaldruck mit Wasserstoff hydriert. Nach 6 Stunden werden nochmals 1,2 g Palladium/Aktivkohle und 60 ml DMF zugegeben. Nach weiteren 19 Stunden wird abermals 1,2 g Palladium/Aktivkohle zugegeben, und das Reaktionsgemisch weitere 15 Stunden gerührt, wobei die Reaktion zum Stillstand kommt. Der totale Verbrauch an Wasserstoff beträgt 4,56 l (88% d.Th.). Die grüngelbe Suspension wird mit 1 l DMF verdünnt, auf 130°C erwärmt und 30 Minuten weitergerührt und durch einen Hartpapierfilter filtriert. Nach dem Nachwaschen des Rückstandes mit 400 ml DMF wird das Filtrat auf ca. 200 ml eingeengt, dann 16 Stunden bei Raumtemperatur stehen gelassen und abfiltriert. Der Rückstand wird mit 200 ml Methanol gewaschen. Man erhält 2,86 g (30% d.Th.) orangefarbenes Pulver der Formel (VIII)

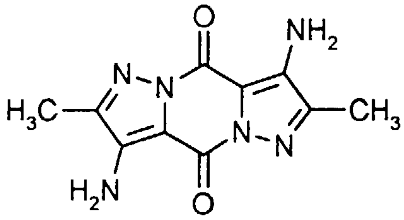

(VIII)

| | | | |
|---|---|---|---|
| und der Elementarzusammensetzung | 48,73% C, | 4,22% H, | 33,42% N; |
| (berechnet für $C_{10}H_{10}N_6O_2$ | 48,78% C, | 4,09% H, | 24,13% N). |

Beispiel 2: 2,46 g der Verbindung der Formel (VIII) aus Beispiel 1 und 2,58 g Barbitursäure werden in 80 ml DMF aufgeschlämmt. In die orange Suspension werden 5 ml Triethylorthoformiat eingerührt; die Reaktionsmischung wird bis auf 130°C aufgeheizt, wobei das bei der Reaktion freiwerdende Ethanol abdestilliert wird. Die nunmehr gelbe Suspension wird noch 21 Stunden bei 130°C gerührt, filtriert und mit 100 ml DMF, 300 ml Methanol und 300 ml Wasser gewaschen. Der noch etwas feuchte Presskuchen wird in 90 ml Dimethylacetamid aufgenommen und 6 Stunden bei 130°C gerührt. Die auf Raumtemperatur abgekühlte Suspension wird abfiltriert und der Rückstand mit 200 ml Methanol und 200 ml Wasser nachgewaschen und getrocknet. Man erhält 3,73 g (71% d.Th.) orangefarbenes Pulver der Formel (IX)

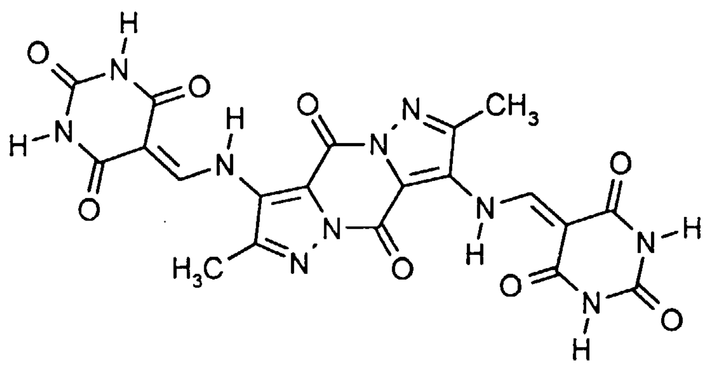

(IX)

| | | | |
|---|---|---|---|
| und der Elementarzusammensetzung | 45,10 % C, | 3,45 % H, | 26,16 % N |
| (berechnet für $C_{20}H_{14}N_{10}O_8 \cdot 0,6\ H_2O$ | 45,05 % C, | 2,87 % H, | 26,27 % N). |

Beispiel 3: 1,29 g der Verbindung der Formel (X),

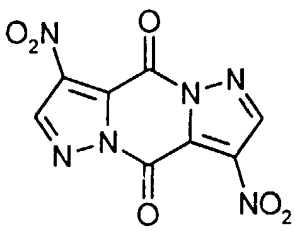

(X)

hergestellt gemäss Baraldi et al., Int. J. Cosmet. Sci. 1995/17, 147-56, werden in 200 ml DMF mit 0,5 g Pd 5% auf Aktivkohle bei 25-35°C unter einem Druck von 15 bar hydriert. Nach 20 Stunden ist die Reaktion beendet, wobei die Wasserstoff-Aufnahme 0,62 l beträgt (100% d.Th.). Das Reaktionsgemisch wird über ein Hartpapier-Filter filtriert und mit 100 ml 60°C warmem DMF nachgewaschen. Das intensiv gelbbraune Filtrat wird am Rotavapor bis auf ein Volumen von ca. 5 ml eingeengt und 2 Tage bei 0-5°C stehengelassen. Die ausgefallenen gelbbraunen, sehr feinen Kristalle werden abfiltriert, mit 10 ml Methanol gewaschen und in 60 ml DMF heiss gelöst. Nach Einengen auf ca. 5 ml und Stehenlassen bei Raumtemperatur während 4 Stunden wird filtriert und der Rückstand getrocknet: Man erhält 105 mg (10% d.Th.) ockerfarbenes Pulver der Formel (XI)

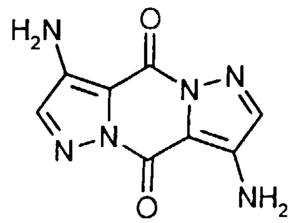

(XI)

| | | | |
|---|---|---|---|
| und der Elementarzusammensetzung | 42,65 % C, | 3,01 % H, | 37,30 % N |
| (berechnet für $C_8H_6N_6O_2 \cdot 0.4\ H_2O$ | 42,63 % C, | 3,04 % H, | 37,29 % N). |

Beispiel 4: 25,64 g der Verbindung der Formel (XII),

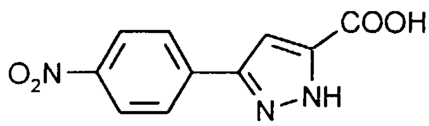

(XII)

hergestellt nach Beilstein E III/IV 25, S. 841, werden in 130 ml Thionylchlorid 17 Stunden unter Rückfluss gerührt. Danach werden ca. 80% des Thionylchlorids abdestilliert und 100 ml Toluol zugegeben. Das Reaktionsgemisch wird filtriert, der Rückstand mit 240 ml Aceton und 300 ml Wasser gewaschen und getrocknet; man erhält 10,07 g (43% d. Th.) hellbraunes Pulver der Formel (XIII)

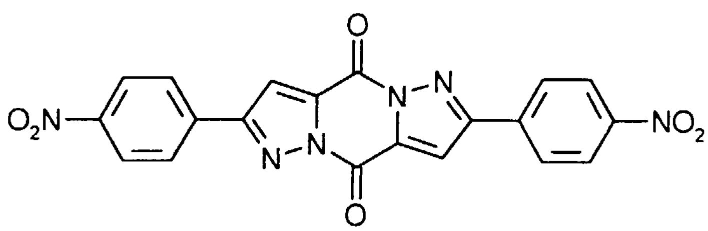

(XIII)

| | | | |
|---|---|---|---|
| und der Elementarzusammensetzung | 55,58 % C, | 2,43 % H, | 19,66 % N |
| (berechnet für $C_{20}H_{10}N_6O_6$ | 55,82 % C, | 2,34 % H, | 19,53 % N). |

10,07 g der so hergestellten Verbindung der Formel (XIII) werden in 500 ml DMF unter Zusatz von 1,0 g Pd 5% auf Aktivkohle bei Raumtemperatur und Normaldruck hydriert. Nach 18 Stunden ist die Reaktion beendet, wobei die Wasserstoff-Aufnahme 3,05 l beträgt (97% d.Th.). Das Reaktionsgemisch wird über ein Hartpapier-Filter filtriert und mit 100 ml DMF nachgewaschen (Falls die hydrierte Verbindung im Reaktionsgemisch bereits ausgefallen ist, muss zuerst mit weiterem DMF verdünnt und vor der Filtration aufgeheizt werden).

Das rotbraune Filtrat wird bis auf ca. 50 ml eingeengt und innert 30 Min. in 2 l Wasser eingerührt. Der rotbraune Niederschlag wird abfiltriert, mit 300 ml Wasser nachgewaschen und getrocknet; man erhält 7,84 g (91% d.Th.) rotbraunes Pulver der Formel (XIV)

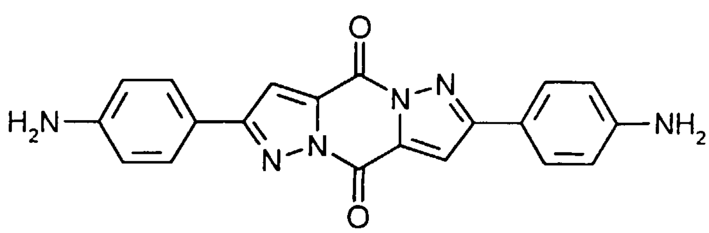

(XIV)

| | | | |
|---|---|---|---|
| und der Elementarzusammensetzung | C 63,50 %, | H 3,92 %, | N 22,33 % |
| (berechnet für $C_{20}H_{14}N_6O_2 \cdot 0{,}4\ H_2O$ | C 63,62 %, | H 3,95 %, | N 22,26 %). |

1,23 g der oben hergestellten Verbindung der Formel (XIV) werden in 60 ml N-Methylpyrrolidon (NMP) aufgeschlämmt, 10 ml Acetanhydrid zugegeben und das Reaktionsgemisch innert 30 Minuten kontinuierlich auf 155°C aufgeheizt, wobei die Farbe der Suspension von rotbraun nach dunkelgelb umschlägt. Nun wird ohne Beheizung noch 45 Min. weiter gerührt, die noch 75°C warme Reaktionsmischung über ein Hartfilter-Papier filtriert, und der Rückstand mit 20 ml NMP und 20 ml Methanol nachgewaschen. Das so erhaltene Rohprodukt wird während einer Stunde in 40 ml siedendem Dimethylacetamid (DMA) rekristallisiert, über ein Hartpapier-Filter abfiltriert und mit 20 ml Ethanol gewaschen. Nach dem Trocknen erhält man 960 mg (78% d.Th.) gelbes Pulver der Formel (XV)

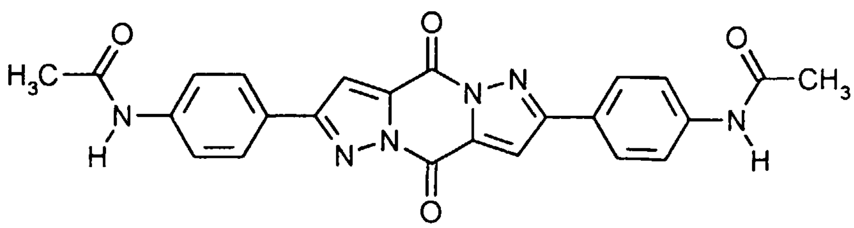

(XV)

| | | | |
|---|---|---|---|
| und der Elementarzusammensetzung | C 61,07 %, | H 5,62 %, | N 17,70 % |
| (berechnet für $C_{24}H_{18}N_6O_4 \cdot H_2O$ | C 61,01 %, | H 4,26 %, | N 17,79 %). |

Das für die Zusammensetzung $C_{24}H_{18}N_6O_4$ errechnete Molekulargewicht von 454 wurde durch ein Massenspektrogramm (DEI) zusätzlich bestätigt.

Beispiel 5: 11,98 g der Verbindung der Formel (XVI),

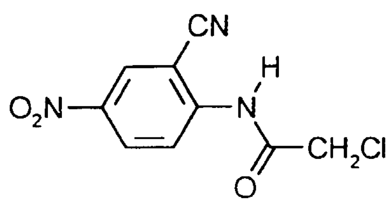

(XVI)

hergestellt nach K. Gewald et al, Chem. Ber. 124, 1237-41 (1991), werden in 40 ml wasserfreiem Diethylenglykoldimethylether (Diglyme) suspendiert, 3,44 g Natrium-tertamylat (Gehalt: 80%) zugegeben und das Reaktionsgemisch auf 85°C aufgeheizt. Man rührt 6 Stunden bei 85°C und danach noch eine Stunde ohne Heizung. Das Reaktionsgemisch wird filtriert, der rotbraune Rückstand mit 200 ml Wasser gewaschen, noch feucht in 80 ml Essigsäure 100% aufgeschlämmt und 30 Minuten bei Raumtemperatur verrührt. Man filtriert, wäscht mit 200 ml Wasser nach und trocknet. Man erhält 3.1 g (31% d.Th.) rotbraunes Pulver der Formel (XVII)

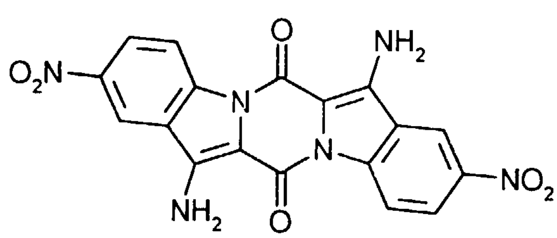

(XVII)

| | | | |
|---|---|---|---|
| und der Elementarzusammensetzung | C 52,29 %, | H 2,71 %, | N 20,24 % |
| (berechnet für $C_{18}H_{10}N_6O_6 \cdot 0,4\ H_2O$ | C 52,28 %, | H 2,63 %, | N 20,32 %). |

Beispiel 6: 30,82 g 2-Amino-4-chlorbenzonitril werden in 450 ml wasserfreiem Toluol aufgeschlämmt und 24,2 ml Chloracetylchlorid zugegeben. Die Reaktionsmischung wird auf -5°C abgekühlt und innert 4 Stunden 49 ml trockenes Triethylamin zugegeben. Man rührt noch 18 Stunden ohne Kühlung bei Raumtemperatur weiter und giesst die Reaktionslösung in 2 l Essigsäureethylester (Essigester). Man schüttelt 3× mit je 700 ml Wasser aus, trocknet die organische Phase mit wasserfreiem Natriumsulfat und reinigt diese Lösung über eine 100 g Kieselgel enthaltende Säule (Nachwaschen mit 500 ml Essigester). Man engt zur Trockne ein, suspendiert in 150 ml Methanol, filtriert und wäscht mit 600 ml Petroläther. Nach dem Trocknen erhält man 31,36 g (68% d.Th.) eines bei 161°C schmelzenden, hellbeigen Pulvers der Formel (XVIII)

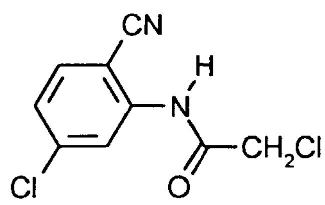

(XVIII)

| | | | | |
|---|---|---|---|---|
| und der Elementarzusammensetzung | C 47,12 %, | H 2,71 %, | N 12,22 %, | Cl 30,78 % |
| (berechnet für $C_9H_6N_2OCl_2$ | C 47,19 %, | H 2,64 %, | N 12,23 %, | Cl 30,95 %). |

9,16 g der oben beschriebenen Verbindung der Formel (XVIII) werden in 40 ml wasserfreiem Diglyme suspendiert, 2,75 g Natrium-tert-butylat (Gehalt: 80%) zugegeben und die Mischung 4 Stunden auf 65°C erhitzt. Man lässt innert einer Stunde auf Raumtemperatur abkühlen, gibt 5 ml Essigsäure 100% zu und rührt noch 17 Stunden bei Raumtemperatur nach. Die gelbe Suspension wird abfiltriert, und der Rückstand mit 200 ml Essigsäure und 400 ml Wasser nachgewaschen. Das Rohprodukt wird 2 Std. in 50 ml DMA rekristallisiert, filtriert, mit 160 ml Methanol und 200 ml Wasser gewaschen und getrocknet; man erhält 4,41 g gelbes Pulver der Formel (XIX)

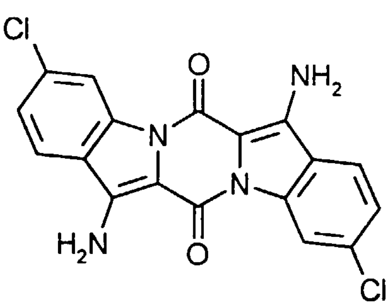

(XIX)

| | | | | |
|---|---|---|---|---|
| und der Elementarzusammensetzung | C 55,85 %, | H 2,75 %, | N 14,52 %, | Cl 18,26 % |
| (berechnet für $C_{18}H_{10}N_4O_2Cl_2$ | C 56,12 %, | H 2,62 %, | N 14,54 %, | Cl 18,41 %). |

2,3 g der oben beschriebenen Verbindung von der Formel (XIX) werden zusammen mit 1,8 ml Acetylchlorid in 100 ml Dimethylacetamid 20 Stunden auf 120°C erhitzt. Die hellgelbe Suspension wird noch eine Stunde ohne Heizung gerührt, filtriert und der Rückstand mit 350 ml Wasser und 150 ml Methanol gewaschen. Nach dem Trocknen erhält man 2,74 g (97%) leuchtgelbe Kristalle der Formel (XX)

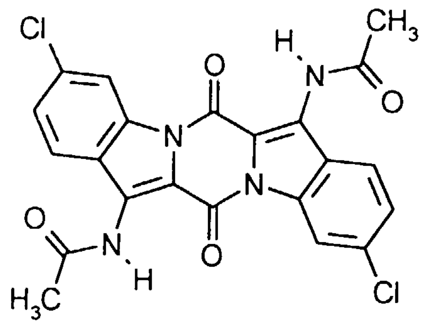

(XX)

| | | | | |
|---|---|---|---|---|
| und der Elementarzusammensetzung | C 56,10 %, | H 3,02 %, | N 11,89 %, | Cl 15,16 % |
| (berechnet für $C_{22}H_{14}N_4O_4Cl_2$ | C 56,31 %, | H 3,01 %, | N 11,94 %, | Cl 15,11 %). |

Beispiel 7: 1,26 g der Verbindung der Formel (XXI)

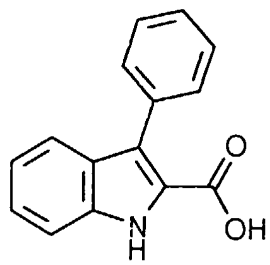

(XXI)

(hergestellt nach Beilstein E II 22, 68) werden in 10 ml Dichloromethan gelöst und unter Zusatz von 0,76 g Thionylchlorid 4 Stunden bei Raumtemperatur und unter Schutzgas gerührt. Das Lösungsmittel und der Überschuss $SOCl_2$ werden abgezogen. Der Rückstand wird in 10 ml Dichloromethan gelöst und 1,0 g Pyridin zugegeben. Das Reaktionsgemisch wird 14 Stunden bei Raumtemperatur gerührt. Es fällt dabei ein gelber Niederschlag aus, der über ein Hartpapierfilter filtriert wird und mit weiteren 10 ml Dichloromethan nachgewaschen wird. Das gelbe Pulver wird während 14 Stunden in 20 ml siedendem Diacetonalkohol nachbehandelt. Man erhält 1,0 g (86% d.Th.) eines gelben Produkts der Formel (XXII)

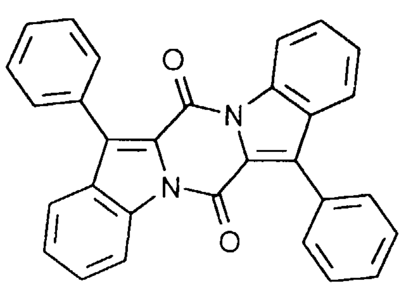

(XXII)

| | | | |
|---|---|---|---|
| und der Elementarzusammensetzung | C 80,95%, | H 4,13%, | N 6,22% |
| (berechnet für $C_{30}H_{18}N_2O_2$ | C 82,18%, | H 4,14%, | N 6,39%). |

Beispiel 8: 3,70 g der Verbindung der Formel (XIV) aus Beispiel 4 und 2,94 g Thiobarbitursäure werden in 150 ml DMF aufgeschlämmt. In die hellbraune Suspension werden 5 ml Triethylorthoformiat eingerührt, und die Reaktionsmischung bis auf 115°C aufgeheizt, wobei das bei der Reaktion freiwerdende Ethanol abdestilliert wird. Die

nunmehr orange Suspension wird noch 20 Stunden bei 115°C gerührt und filtriert, und der Rückstand wird mit 100 ml Methanol gewaschen. Der noch etwas feuchte Presskuchen wird in 150 ml DMA aufgenommen und 20 Stunden bei 120°C gerührt. Die auf Raumtemperatur abgekühlte Suspension wird abfiltriert, und der Rückstand mit 240 ml Methanol und 300 ml Wasser nachgewaschen und getrocknet. Man erhält 5,75 g (85% d.Th.) eines orangefarbenen Pulvers der Formel (XXIII)

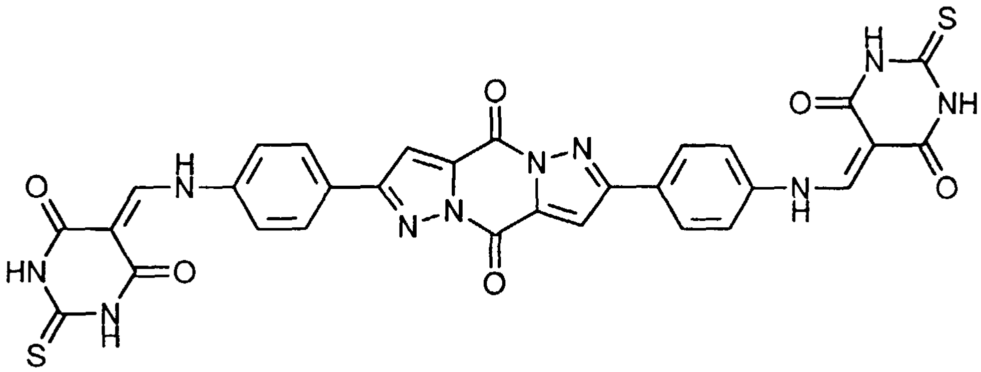

(XXIII)

| | |
|---|---|
| und der Elementarzusammensetzung | C 51,28%, H 3,12%, N 19,90%, S 8,02% |
| (berechnet für $C_{30}H_{18}N_{10}O_6S_2 \cdot 1{,}4H_2O$ | C 51,19%, H 2,98%, N 19,90%, S 9,11%). |

Beispiele 9-24: Jeweils wird 1 Gewichtsteil der angegebenen Verbindung aus dem angegebenen Beispiel in 100 Gewichtsteile des angegebenen Polymers eingearbeitet. Man erhält brillante Färbungen der angegebenen Farbe mit den angegebenen Echtheiten (PVC = Polyvinylchlorid, HDPE = Polyethylen hoher Dichte):

| Beispiel Nr. | Verbindung | | Polymer | erhaltene Farbe | erhaltene Echtheiten |
|---|---|---|---|---|---|
| | der Formel | aus Beispiel | | | |
| 9 | (VIII) | 1 | PVC | orangegelb | sehr gut |
| 10 | (VIII) | 1 | HDPE | orangegelb | sehr gut |
| 11 | (IX) | 2 | PVC | gelb-orange †,‡ | gut |
| 12 | (IX) | 2 | HDPE | gelb-orange †,‡ | gut |
| 13 | (XI) | 3 | PVC | gelb ‡ | sehr gut |
| 14 | (XI) | 3 | HDPE | gelb ‡ | sehr gut |
| 15 | (XV) | 4 | PVC | gelb | sehr gut |
| 16 | (XV) | 4 | HDPE | gelb | sehr gut |
| 17 | (XVII) | 5 | PVC | orange | sehr gut |
| 18 | (XVII) | 5 | HDPE | orange | sehr gut |
| 19 | (XX) | 6 | PVC | grünstichig gelb † | gut |
| 20 | (XX) | 6 | HDPE | grünstichig gelb † | gut |
| 21 | (XXII) | 7 | PVC | grünstichig gelb | gut |
| 22 | (XXII) | 7 | HDPE | grünstichig gelb | gut |
| 23 | (XXIII) | 8 | PVC | gelb | gut |
| 24 | (XXIII) | 8 | HDPE | gelb | gut |

† diese Färbungen zeichnen sich durch eine besonders hohe Brillanz aus

‡ diese Färbungen zeichnen sich durch eine besonders hohe Farbstärke aus

## Patentansprüche

**1.** Verfahren zum Färben von hochmolekularem organischem Material in der Masse, gekennzeichnet durch die Verwendung einer Verbindung der Formel (I)

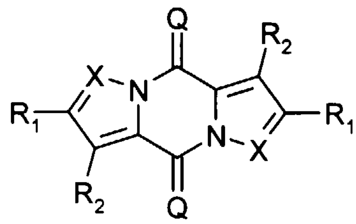
(I),

worin Q für O oder S, und X für $C(R_3)$ oder N stehen,

$R_1$, $R_2$ und $R_3$ unabhängig voneinander für Wasserstoff, Halogen ausgewählt aus der Gruppe bestehend aus Chlor, Brom und Fluor, $C_1$-$C_6$Alkyl, $C_1$-$C_6$Alkoxy, Hydroxy, Cyan, Nitro, Amino, Morpholino,

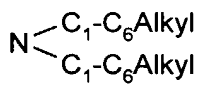
,

Halogen-$C_1$-$C_6$alkyl, NH-$E_1$, $A_1$ oder

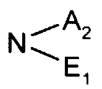

stehen,

worin $A_1$ und $A_2$ unabhängig voneinander mit 3 Resten $R_4$, $R_5$ und $R_6$ substituiertes $C_6$-$C_{12}$Aryl, oder mit 3 Resten $R_4$, $R_5$ und $R_6$ substituiertes $C_3$-$C_{12}$Heteroaryl, welches 1 bis 3 Heteroatome, ausgewählt aus der Gruppe bestehend aus N, O und S, im Ringsystem enthält, bedeuten,

und $E_1$ für $C_1$-$C_6$Alkyl,

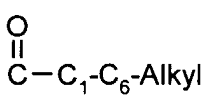
, $A_1$, 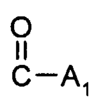 oder 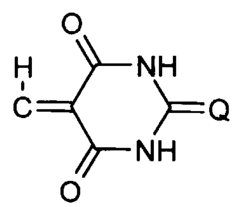

steht,

oder $R_1$ und $R_2$ zusammen oder $R_1$ und $R_3$ zusammen einen Rest $A_3$ darstellen,

worin $A_3$ mit 3 Resten $R_7$, $R_8$ und $R_9$ substituiertes $C_4$-$C_8$Alkenylen oder $C_8$-$C_{12}$Aralkenylen bedeutet,

und $R_4$ bis $R_9$ unabhängig voneinander für Wasserstoff, Halogen, ausgewählt aus der Gruppe bestehend aus Chlor, Brom und Fluor, $C_1$-$C_6$Alkyl, $C_1$-$C_6$Alkoxy, Hydroxy, Cyan, Nitro, Amino, Morpholino, NH-$C_1$-$C_6$Alkyl,

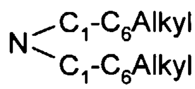
,

Halogen-$C_1$-$C_6$alkyl,

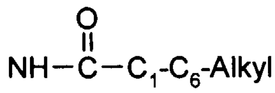, 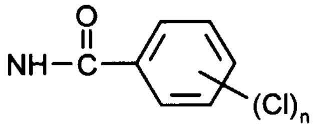 oder 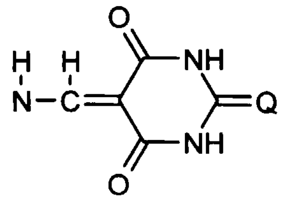

stehen,
worin n Null oder eine ganze Zahl von 1 bis 3 ist.

**2.** Verfahren gemäss Anspruch 1, worin in Formel (I) Q für O steht.

**3.** Verfahren gemäss Anspruch 1, worin X für N steht, und $R_1$ und $R_2$ $C_1$-$C_4$Alkyl, $C_1$-$C_4$Alkoxy, Amino, $A_1$ oder NH-$E_1$ bedeuten.

**4.** Verfahren gemäss Anspruch 3, worin $A_1$ mit 3 Resten $R_{10}$, $R_{11}$ und $R_{12}$ substituiertes $C_6$-$C_{12}$Aryl bedeutet, wobei $R_{10}$ bis $R_{12}$ unabhängig voneinander für $R_4$ bis $R_6$ nach Anspruch 1 stehen.

**5.** Verfahren gemäss Anspruch 4, worin $R_{10}$ bis $R_{12}$ für Wasserstoff, $C_1$-$C_6$Alkoxy, Amino,

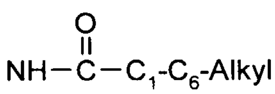, 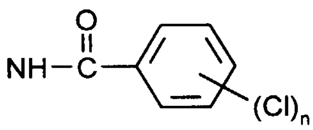 oder 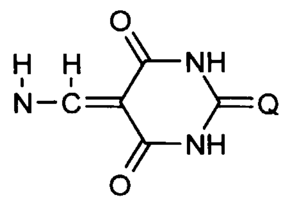

stehen.

**6.** Verfahren gemäss Anspruch 1, worin X für $C(R_3)$ steht, ein Rest $R_1$ oder $R_2$ $C_1$-$C_4$Alkyl, $C_1$-$C_4$Alkoxy, Amino, $A_1$ oder NH-$E_1$ bedeutet, und der andere Rest $R_2$ oder $R_1$ zusammen mit $R_3$ einen Rest

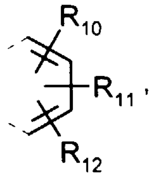, 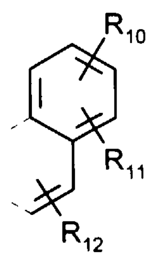 oder 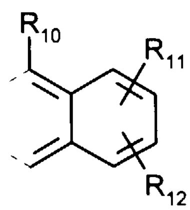

bildet,
wobei $R_{10}$ bis $R_{12}$ die im Anspruch 4 gegebene Definition haben.

**7.** Verfahren gemäss Anspruch 6, worin $R_1$ zusammen mit $R_3$ einen Rest

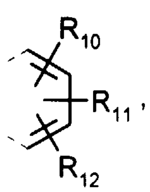

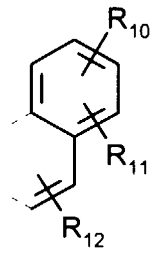 oder 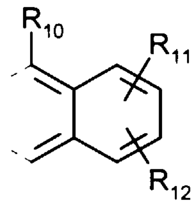 .

**8.** Verfahren gemäss Anspruch 1, worin

1] X für N steht, $R_1$ Methyl, Ethyl oder $A_1$ ist, und $R_2$ Amino oder NH-$E_1$ bedeutet;

2] X für N steht, $R_1$ ein Rest $A_1$ ist, und $R_2$ Wasserstoff bedeutet;

3] X für N steht, $R_1$ Wasserstoff ist, und $R_2$ Amino, Morpholino,

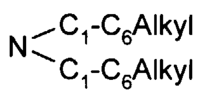 ,

NH-$E_1$ oder

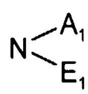

bedeutet; oder

4] X für C($R_{13}$) steht, $R_1$ und $R_2$ zusammen einen Rest

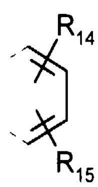

sind, $R_{13}$ Amino oder NH-$E_1$ bedeutet, und $R_{14}$ sowie $R_{15}$ unabhängig voneinander für Wasserstoff, Amino, NH-$E_1$ oder Nitro stehen.

**9.** Verfahren gemäss Anspruch 1, worin die Verbindung der Formel (I) in mindestens einer tautomeren Form mindestens zwei je an einem Stickstoff gebundene Wasserstoffe aufweist.

**10.** Verfahren gemäss Anspruch 1, worin die Verbindung der Formel (I) eine spezifische Oberfläche nach BET von 5-150 $m^2$/g aufweist.

**11.** Verfahren gemäss Anspruch 1, worin die Verbindung der Formel (I) in einer Menge von 0,01 bis 30 Gew.-%, vorzugsweise 0,1 bis 10 Gew.-%, bezogen auf das zu färbende hochmolekulare organische Material, eingesetzt wird.

**12.** Verbindung der Formel

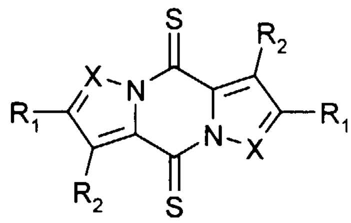 (IV),

worin X, $R_1$ und $R_2$ die gleiche Definition haben, als in Formel (I) gemäss Anspruch 1.

**13.** Verbindung der Formel

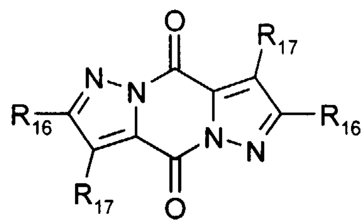 (V),

worin

- ♦ $R_{16}$ $C_6$-$C_{12}$Aryl bedeutet, welches mindestens mit einem Rest $C_1$-$C_6$Alkoxy, Amino, Morpholino, NH-$C_1$-$C_6$Alkyl,

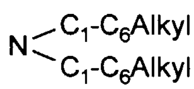 , NH—$E_2$ oder 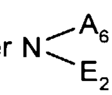

substituiert ist, und

$R_{17}$ unabhängig von $R_{16}$ für Wasserstoff, Chlor, Brom, Fluor, $C_1$-$C_6$Alkyl, $C_1$-$C_6$Alkoxy, Hydroxy, Cyan, Nitro, Amino, Morpholino,

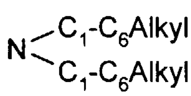 ,

Halogen-$C_1$-$C_6$alkyl, NH-$E_2$, $A_5$ oder

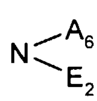

steht,

- mit der Massgabe, dass $R_{17}$ nicht Wasserstoff ist, wenn $R_{16}$ eine Gruppe

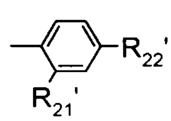

ist, worin $R_{21}$' für Alkoxy und $R_{22}$' für Wasserstoff, Alkyl, Alkoxy, Halogen oder Hydroxy, oder $R_{22}$' für

Alkoxy und $R_{21}$' für Wasserstoff, Alkyl, Alkoxy, Halogen oder Hydroxy stehen;

oder

- ♦ $R_{16}$ Wasserstoff, Chlor, Brom, Fluor, $C_1$-$C_6$Alkyl, $C_1$-$C_6$Alkoxy, Hydroxy, Cyan, Nitro, Amino, Morpholino

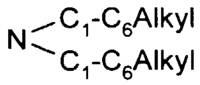, Halogen-$C_1$-$C_6$alkyl, NH—$E_2$, $A_5$ oder 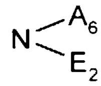

bedeutet, und

$R_{17}$ unabhängig von $R_{16}$ für $C_1$-$C_6$Alkoxy, Amino, Morpholino,

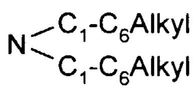,

NH-$E_2$,

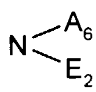

oder A5 steht;
oder

- ♦ $R_{16}$ und $R_{17}$ zusammen einen Rest $A_7$ darstellen,
wobei

| | |
|---|---|
| $E_2$ | für $C_1$-$C_6$Alkyl, 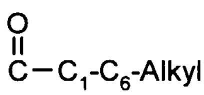, $A_5$, 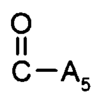 oder 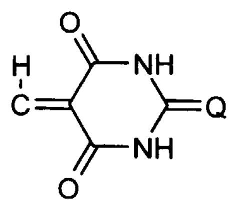 steht, |
| $A_5$ und $A_6$ | unabhängig voneinander mit 3 Resten $R_{21}$, $R_{22}$ und $R_{23}$ substituiertes $C_6$-$C_{12}$Aryl, oder mit 3 Resten $R_{21}$, $R_{22}$ und $R_{23}$ substituiertes $C_3$-$C_{12}$Heteroaryl, welches 1 bis 3 Heteroatome, ausgewählt aus der Gruppe bestehend aus N, O und S, im Ringsystem enthält, bedeuten, |
| $A_7$ | mit 3 Resten $R_{24}$, $R_{25}$ und $R_{27}$ substituiertes $C_4$-$C_8$Alkenylen oder $C_8$-$C_{12}$Aralkenylen bedeutet, |
| $R_{21}$ bis $R_{25}$ | unabhängig voneinander für Wasserstoff, Chlor, Brom, Fluor, $C_1$-$C_6$Alkyl, $C_1$-$C_6$Alkoxy, Hydroxy, Cyan, Nitro, Amino, Morpholino, NH-$C_1$-$C_6$Alkyl, |

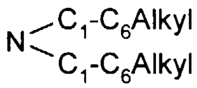 , Halogen-$C_1$-$C_6$alkyl, 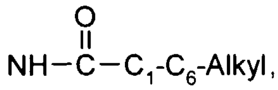 , 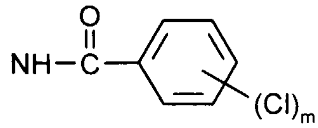

oder

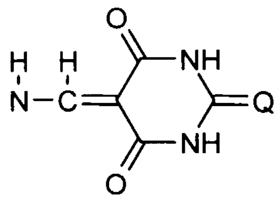

stehen, worin m Null oder eine ganze Zahl von 1 bis 3 ist;

$R_{27}$ für $C_1$-$C_6$Alkoxy steht, und

Q gleich O oder S ist.

**14.** Verbindung der Formel

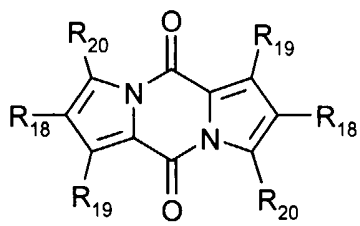 (VI),

worin

- ♦ $R_{18}$ für Wasserstoff, Chlor, Brom, Fluor, $C_1$-$C_6$Alkyl, $C_1$-$C_6$Alkoxy, Hydroxy, Cyan, Nitro, Amino, Morpholino,

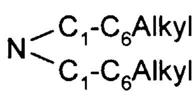 , Halogen-$C_1$-$C_6$alkyl, NH—$E_3$, $A_8$ oder 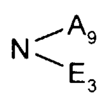,

$R_{19}$ für $C_1$-$C_6$Alkoxy, Amino, Morpholino, NH-$C_1$-$C_6$Alkyl,

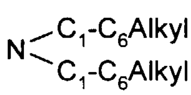 ,

NH-$E_3$, $A_8$ oder

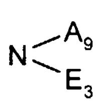,

und

$R_{20}$ für Wasserstoff, Chlor, Brom, Fluor, $C_1$-$C_6$Alkyl, $C_1$-$C_6$Alkoxy, Hydroxy, Cyan, Nitro, Amino, Morpholino,

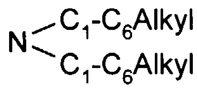 , Halogen-$C_1$-$C_6$alkyl, NH—$E_3$ oder 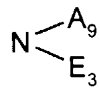

stehen;
oder

- $R_{18}$ für $C_1$-$C_6$Alkoxy, Amino, Morpholino, NH-$C_1$-$C_6$Alkyl,

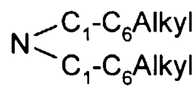 ,

NH-$E_3$ oder

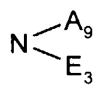

steht,
$R_{19}$ für Wasserstoff, Chlor, Brom, Fluor, $C_1$-$C_6$Alkyl, $C_1$-$C_6$Alkoxy, Hydroxy, Cyan, Nitro, Amino, Morpholino,

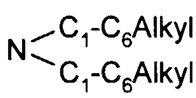 , Halogen-$C_1$-$C_6$alkyl, NH—$E_3$, $A_8$ oder 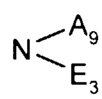

steht, und
$R_{20}$ ein Rest $A_8$ ist;
oder

- $R_{18}$ und $R_{19}$ unabhängig voneinander für Wasserstoff, Chlor, Brom, Fluor, $C_1$-$C_6$Alkyl, $C_1$-$C_6$Alkoxy, Hydroxy, Cyan, Nitro, Amino, Morpholino,

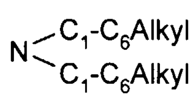 ,

Halogen-$C_1$-$C_6$alkyl, NH-$E_3$, $A_8$ oder

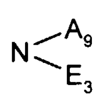

stehen, und
$R_{20}$ ein Rest $A_{12}$ ist;
oder

- $R_{18}$ und $R_{19}$ zusammen einen Rest $A_{11}$ darstellen, und

$R_{20}$ für Wasserstoff, Halogen, Chlor, Brom, Fluor, $C_1$-$C_6$Alkyl, $C_1$-$C_6$Alkoxy, Hydroxy, Cyan, Nitro, Amino, Morpholino,

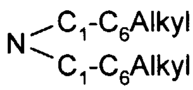 ,

Halogen-$C_1$-$C_6$alkyl, NH-$E_3$, $A_8$ oder

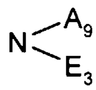

steht,
oder

- ♦ $R_{18}$ und $R_{20}$ zusammen einen Rest $A_{11}$ darstellen, und
  $R_{19}$ für $C_1$-$C_6$Alkyl oder Amino steht,
  oder

- ♦ $R_{18}$ und $R_{20}$ zusammen einen Rest $A_{10}$ darstellen, und
  $R_{19}$ für Chlor, Brom, Fluor, $C_1$-$C_6$Alkoxy, Hydroxy, Cyan, Nitro, Morpholino,

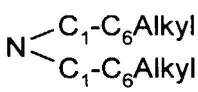 , Halogen-$C_1$-$C_6$alkyl, NH—$E_3$, $A_8$ oder 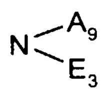

steht,

- mit der Massgabe, dass es sich bei der Verbindung der Formel (VI) nicht um eine Verbindung der Formel

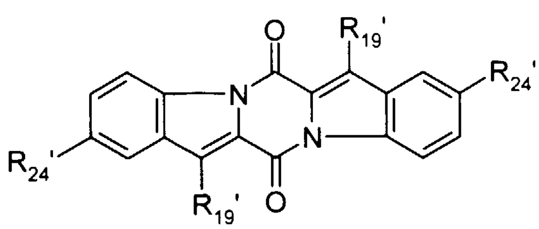 (XXIV)

handelt,
worin wenn $R_{24}$' gleich Wasserstoff, $R_{19}$' gleich Cyano oder Hydroxy, oder wenn $R_{24}$' gleich Chlor, $R_{19}$' gleich Phenyl, o-Chlor-phenyl oder o-Fluor-phenyl sind;

worin

$E_3$ für $C_1$-$C_6$Alkyl,

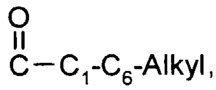, $A_8$, 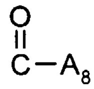 oder 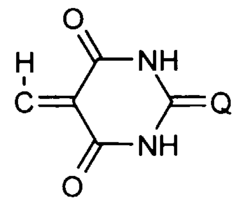

steht;

| | |
|---|---|
| $A_8$ und $A_9$ | unabhängig voneinander mit 3 Resten $R_{21}$, $R_{22}$ und $R_{23}$ substituiertes $C_6$-$C_{12}$Aryl, oder mit 3 Resten $R_{21}$, $R_{22}$ und $R_{23}$ substituiertes $C_3$-$C_{12}$Heteroaryl, welches 1 bis 3 Heteroatome, ausgewählt aus der Gruppe bestehend aus N, O und S, im Ringsystem enthält, bedeuten; |
| $A_{10}$ | mit 3 Resten $R_{24}$, $R_{25}$ und $R_{26}$ substituiertes $C_4$-$C_8$Alkenylen oder $C_8$-$C_{12}$Arylen bedeutet; |
| $A_{11}$ | mit 3 Resten $R_{24}$, $R_{25}$ und $R_{27}$ substituiertes $C_4$-$C_8$Alkenylen oder $C_8$-$C_{12}$Arylen bedeutet, |
| $A_{12}$ | mit 3 Resten $R_{21}$, $R_{22}$ und $R_{28}$ substituiertes $C_6$-$C_{12}$Aryl, oder mit 3 Resten $R_{21}$, $R_{22}$ und $R_{28}$ substituiertes $C_3$-$C_{12}$Heteroaryl, welches 1 bis 3 Heteroatome, ausgewählt aus der Gruppe bestehend aus N, O und S, im Ringsystem enthält, bedeutet; |
| $R_{21}$ bis $R_{26}$ | unabhängig voneinander für Wasserstoff, Chlor, Brom, Fluor, $C_1$-$C_6$Alkyl, $C_1$-$C_6$Alkoxy, Hydroxy, Cyan, Nitro, Amino, Morpholino, NH-$C_1$-$C_6$Alkyl, |

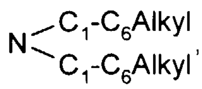,

Halogen-$C_1$-$C_6$alkyl,

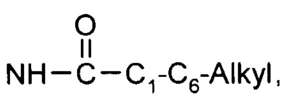, 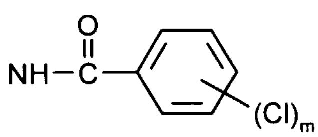 oder 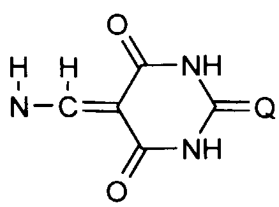

stehen;

| | |
|---|---|
| $R_{27}$ und $R_{28}$ | unabhängig voneinander für $C_1$-$C_6$Alkoxy, Amino, Morpholino, NH-$C_1$-$C_6$Alkyl, |

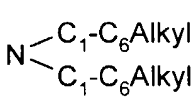, NH—$E_3$ oder 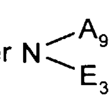

stehen;

| | |
|---|---|
| m | Null oder eine ganze Zahl von 1 bis 3 ist; und |
| Q | für O oder S steht. |

**15.** Verbindung nach Anspruch 12, 13 oder 14, welche mindestens eine Gruppe $C_1$-$C_6$Alkoxy, Amino, Morpholino,

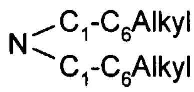, $NH—E_2$ oder 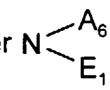

enthält, worin $E_1$, $E_2$ und $A_6$ der in Anspruch 12 gegebener Definition entsprechen.

**16.** Stoffzusammensetzung, enthaltend eine Verbindung der Formel (I) gemäss Anspruch 1 und ein hochmolekulares organisches Material.

**Claims**

**1.** A process for bulk coloration of high molecular weight organic material, which comprises the use of a compound of the formula (I)

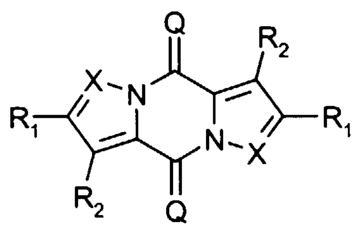

(I),

in which Q is O or S, and X is $C(R_3)$ or N,
$R_1$, $R_2$ and $R_3$ independently of one another are hydrogen, halogen selected from the group consisting of chlorine, bromine and fluorine, $C_1$-$C_6$alkyl, $C_1$-$C_6$alkoxy, hydroxyl, cyano, nitro, amino, morpholino,

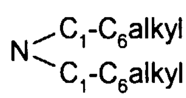,

halo- $C_1$-$C_6$alkyl, NH-$E_1$, $A_1$ or

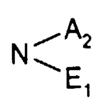,

in which $A_1$ and $A_2$ independently of one another are $C_6$-$C_{12}$aryl substituted by 3 radicals $R_4$, $R_5$ and $R_6$, or $C_3$-$C_{12}$heteroaryl which is substituted by 3 radicals $R_4$, $R_5$ and $R_6$ and contains, in the ring system, 1 to 3 heteroatoms selected from the group consisting of N, O and S,
and $E_1$ is $C_1$-$C_6$alkyl,

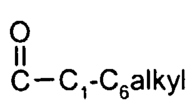, $A_1$, 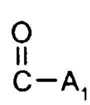

or

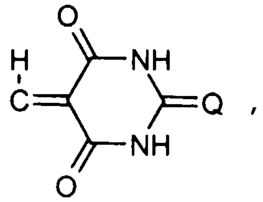

or $R_1$ and $R_2$ together, or $R_1$ and $R_3$ together, are a radical $A_3$,

in which $A_3$ is $C_4$-$C_8$alkenylene or $C_8$-$C_{12}$aralkenylene which are substituted by 3 radicals $R_7$, $R_8$ and $R_9$, and $R_4$ to $R_9$ independently of one another are hydrogen, halogen selected from the group consisting of chlorine, bromine and fluorine, $C_1$-$C_6$alkyl, $C_1$-$C_6$alkoxy, hydroxyl, cyano, nitro, amino, morpholino, NH-$C_1$-$C_6$alkyl,

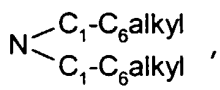

halo-$C_1$-$C_6$alkyl,

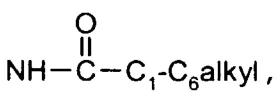 , 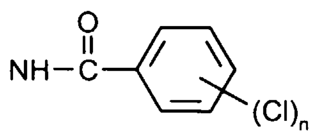 or 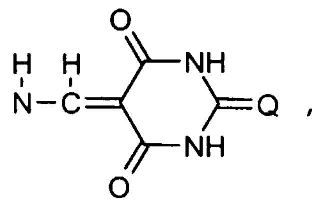 ,

in which n is zero or an integer from 1 to 3.

**2.** A process according to claim 1, wherein, in formula (I), Q is O.

**3.** A process according to claim 1, wherein X is N and $R_1$ and $R_2$ are $C_1$-$C_4$alkyl, $C_1$-$C_4$alkoxy, amino, $A_1$ or NH-$E_1$.

**4.** A process according to claim 3, wherein $A_1$ is $C_6$-$C_{12}$aryl substituted by 3 radicals $R_{10}$, $R_{11}$ and $R_{12}$, in which $R_{10}$ to $R_{12}$ independently of one another are $R_4$ to $R_6$ according to claim 1.

**5.** A process according to claim 4, wherein $R_{10}$ to $R_{12}$ are hydrogen, $C_1$-$C_6$alkoxy, amino,

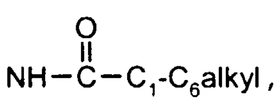 , 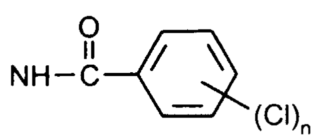 or 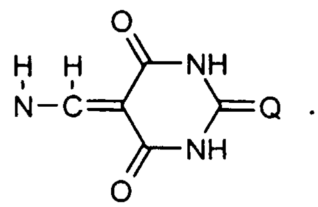 .

**6.** A process according to claim 1, wherein X is $C(R_3)$, one radical $R_1$ or $R_2$ is $C_1$-$C_4$alkyl, $C_1$-$C_4$alkoxy, amino, $A_1$ or NH-$E_1$, and the other radical $R_2$ or $R_1$ together with $R_3$ forms a radical

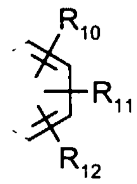, 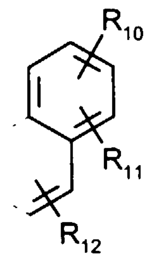 or 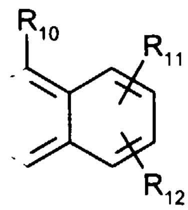,

in which $R_{10}$ to $R_{12}$ are as defined in claim 4.

**7.** A process according to claim 6, in which $R_1$, together with $R_3$, forms a radical

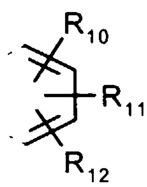, 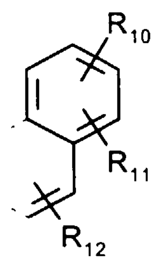 or 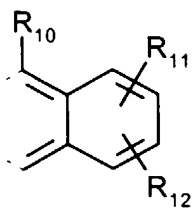.

**8.** A process according to claim 1, wherein

1] X is N, $R_1$ is methyl, ethyl or $A_1$ and $R_2$ is amino or NH-$E_1$;

2] X is N, $R_1$ is a radical $A_1$ and $R_2$ is hydrogen;

3] X is N, $R_1$ is hydrogen and $R_2$ is amino, morpholino,

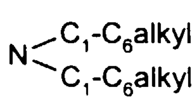, NH—$E_1$ or 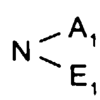;

or

4] X is C($R_{13}$), $R_1$ and $R_2$ together are a radical

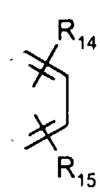,

$R_{13}$ is amino or NH-$E_1$ and $R_{14}$ and $R_{15}$ independently of one another are hydrogen, amino, NH-$E_1$ or nitro.

**9.** A process according to claim 1, wherein the compound of the formula (I) has at least two hydrogens each bonded to a nitrogen in at least one tautomeric form.

**10.** A process according to claim 1, wherein the compound of the formula (I) has a specific BET surface area of 5-150 $m^2$/g.

**11.** A process according to claim 1, wherein the compound of the formula (I) is employed in an amount of 0.01 to 30% by weight, preferably 0.1 to 10% by weight, based on the high molecular weight organic material to be coloured.

**12.** A compound of the formula

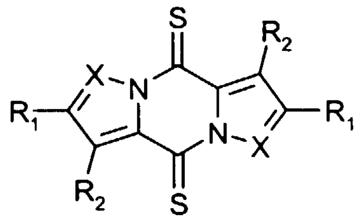

(IV)

in which X, $R_1$ and $R_2$ have the same definition as in formula (I) according to claim 1.

**13.** A compound of the formula

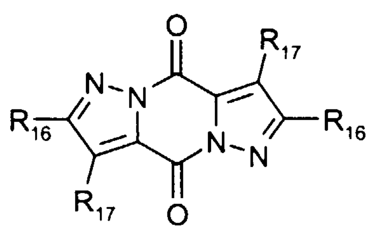

(V)

in which

♦ $R_{16}$ is $C_6$-$C_{12}$aryl substituted at least by a radical $C_1$-$C_6$alkoxy, amino, morpholino, NH-$C_1$-$C_6$alkyl,

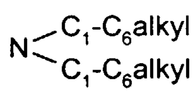, NH-$E_2$ or 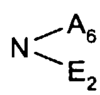,

and

$R_{17}$ independently of $R_{16}$ is hydrogen, chlorine, bromine, fluorine, $C_1$-$C_6$alkyl, $C_1$-$C_6$alkoxy, hydroxyl, cyano, nitro, amino, morpholino,

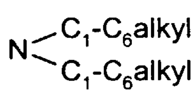, halo-$C_1$-$C_6$alkyl, NH-$E_2$, $A_5$ or 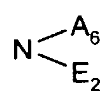,

- with the proviso that $R_{17}$ is not hydrogen if $R_{16}$ is a group

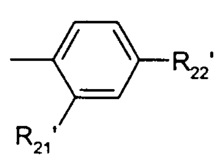

in which $R_{21}$' is alkoxy and $R_{22}$' is hydrogen, alkyl, alkoxy, halogen or hydroxyl or $R_{22}$' is alkoxy and $R_{21}$' is hydrogen, alkyl, alkoxy, halogen or hydroxyl;

or

- $R_{16}$ is hydrogen, chlorine, bromine, chlorine, fluorine, $C_1$-$C_6$alkyl,$C_1$-$C_6$ alkoxy, hydroxyl, cyano, nitro, amino, morpholino,

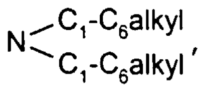

halo-$C_1$-$C_6$alkyl, NH-$E_2$, $A_5$ or

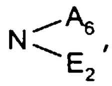

and

$R_{17}$ independently of $R_{16}$ is $C_1$-$C_6$alkoxy, amino, morpholino,

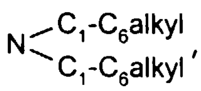 NH-$E_2$, 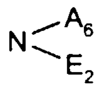

or $A_5$ ; or

- $R_{16}$ and $R_{17}$ together are a radical $A_7$,

where

$E_2$ is $C_1$-$C_6$alkyl,

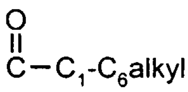 $A_5$, 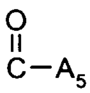 or 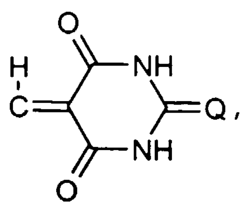

$A_5$ and $A_6$ independently of one another are $C_6$-$C_{12}$aryl substituted by 3 radicals $R_{21}$, $R_{22}$ and $R_{23}$, or $C_3$-$C_{12}$heteroaryl substituted by 3 radicals $R_{21}$, $R_{22}$ and $R_{23}$ and containing 1 to 3 heteroatoms selected from the group consisting of N, O and S in the ring system,

$A_7$ is $C_8$-$C_{12}$aralkenylene or $C_4$-$C_8$alkenylene substituted by 3 radicals $R_{24}$, $R_{25}$ and $R_{27}$,

$R_{21}$ to $R_{25}$ independently of one another are hydrogen, chlorine, bromine, fluorine, $C_1$-$C_6$alkyl, $C_1$-$C_6$alkoxy, hydroxyl, cyano, nitro, amino, morpholino, NH-$C_1$-$C_6$alkyl,

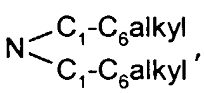 halo-$C_1$-$C_6$alkyl, 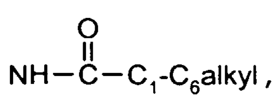

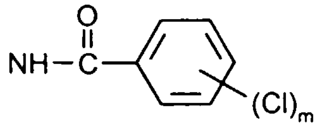 or 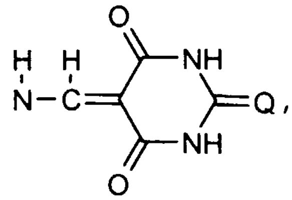

in which m is zero or an integer from 1 to 3;

$R_{27}$ is $C_1$-$C_6$alkoxy, and

Q is O or S.

**14.** A compound of the formula

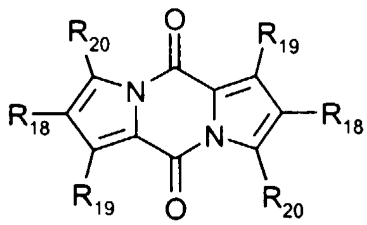 (VI)

in which

- $R_{18}$ is hydrogen, chlorine, bromine, fluorine, $C_1$-$C_6$alkyl, $C_1$-$C_6$alkoxy, hydroxyl, cyano, nitro, amino, morpholino,

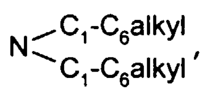

halo-$C_1$-$C_6$alkyl, NH-$E_3$, $A_8$ or

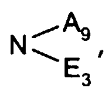

$R_{19}$ is $C_1$-$C_6$alkoxy, amino, morpholino, NH-$C_1$-$C_6$alkyl,

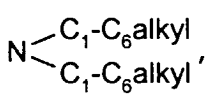 NH-$E_3$, $A_8$ or 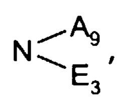

and

$R_{20}$ is hydrogen, chlorine, bromine, fluorine, $C_1$-$C_6$alkyl, $C_1$-$C_6$alkoxy, hydroxyl, cyano, nitro, amino, morpholino,

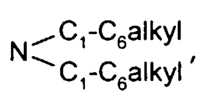

halo-$C_1$-$C_6$alkyl, NH-$E_3$ or

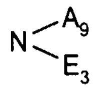;

or

- ♦ $R_{18}$ is $C_1$-$C_6$alkoxy, amino, morpholino, NH-$C_1$-$C_6$alkyl,

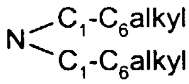, NH-$E_3$ or 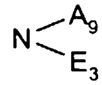,

$R_{19}$ is hydrogen, chlorine, bromine, fluorine, $C_1$-$C_6$alkyl, $C_1$-$C_6$alkoxy, hydroxyl, cyano, nitro, amino, morpholino,

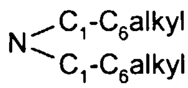,

halo-$C_1$-$C_6$alkyl, NH-$E_3$, $A_8$ or

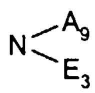,

and
$R_{20}$ is a radical $A_8$;
or

- ♦ $R_{18}$ and $R_{19}$ independently of one another are hydrogen, chlorine, bromine, fluorine, $C_1$-$C_6$alkyl, $C_1$-$C_6$ alkoxy, hydroxyl, cyano, nitro, amino, morpholino,

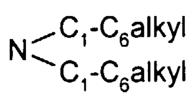,

halo-$C_1$-$C_6$alkyl, NH-$E_3$, $A_8$ or

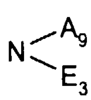,

and
$R_{20}$ is a radical $A_{12}$;
or

- ♦ $R_{18}$ and $R_{19}$ together are a radical $A_{11}$, and
$R_{20}$ is hydrogen, halogen, chlorine, bromine, fluorine, $C_1$-$C_6$alkyl, $C_1$-$C_6$alkoxy, hydroxyl, cyano, nitro, amino, morpholino,

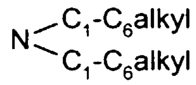

halo-$C_1$-$C_6$-alkyl, NH-$E_3$, $A_8$ or

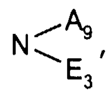

or

- ♦ $R_{18}$ and $R_{20}$ together are a radical $A_{11}$, and
  $R_{19}$ is $c_1$-$c_6$alkyl or amino,

or

- ♦ $R_{18}$ and $R_{20}$ together are a radical $A_{10}$, and
  $R_{19}$ is chlorine, bromine, fluorine, $C_1$-$C_6$alkoxy, hydroxyl, cyano, nitro, morpholino,

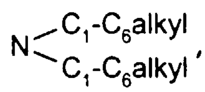

halo-$C_1$-$C_6$alkyl, NH-$E_3$, $A_8$ or

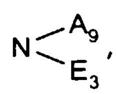

- with the proviso that the compound of the formula (VI) is not a compound of the formula

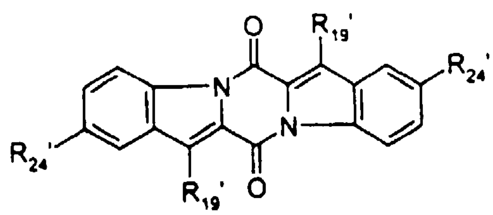 (XXIV)

in which if $R_{24}$' is hydrogen, $R_{19}$' is cyano or hydroxyl, or if $R_{24}$' is chlorine, $R_{19}$' is phenyl, o-chlorophenyl or o-fluorophenyl;

in which

$E_3$ is $C_1$-$C_6$alkyl,

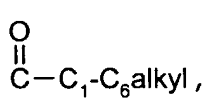 $A_8$, 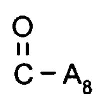

or

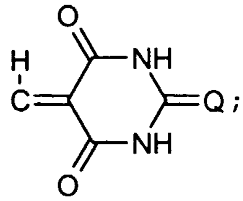

| | |
|---|---|
| $A_8$ and $A_9$ | independently of one another are $C_6$-$C_{12}$aryl substituted by 3 radicals $R_{21}$, $R_{22}$ and $R_{23}$, or $C_3$-$C_{12}$heteroaryl substituted by 3 radicals $R_{21}$, $R_{22}$ and $R_{23}$ and containing 1 to 3 heteroatoms selected from the group consisting of N, 0 and S in the ring system; |
| $A_{10}$ | is $C_8$-$C_{12}$arylene or $C_4$-$C_8$alkenylene substituted by 3 radicals $R_{24}$, $R_{25}$ and $R_{26}$; |
| $A_{11}$ | is $C_8$-$C_{12}$arylene or $C_4$-$C_8$alkenylene substituted by 3 radicals $R_{24}$, $R_{25}$ and $R_{27}$, |
| $A_{12}$ | is $C_6$-$C_{12}$aryl substituted by 3 radicals $R_{21}$, $R_{22}$ and $R_{28}$, or $C_3$-$C_{12}$heteroaryl substituted by 3 radicals $R_{21}$, $R_{22}$ and $R_{28}$ and containing 1 to 3 heteroatoms selected from the group consisting of N, O and S in the ring system; |
| $R_{21}$ to $R_{26}$ | independently of one another are hydrogen, chlorine, bromine, fluorine, $C_1$-$C_6$alkyl, $C_1$-$C_6$alkoxy, hydroxyl, cyano, nitro, amino, morpholino, NH-$C_1$-$C_6$alkyl, 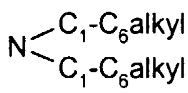, halo-$C_1$-$C_6$alkyl, 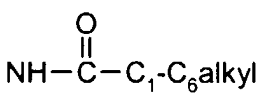, 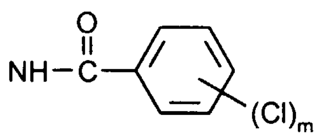 or 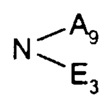; |
| $R_{27}$ and $R_{28}$ | independently of one another are $C_1$-$C_6$alkoxy, amino, morpholino, NH-$C_1$-$C_6$alkyl, 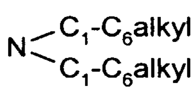, NH-$E_3$ or 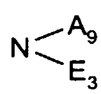; |
| m | is zero or an integer from 1 to 3; and |
| Q | is O or S. |

**15.** A compound according to claim 12, 13 or 14, which contains at least one group from the series consisting of $C_1$-$C_6$alkoxy, amino, morpholino,

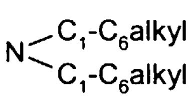, NH—$E_2$ and 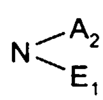

in which $E_1$, $E_2$ and $A_6$ are as defined in claim 12.

**16.** A composition of substances comprising a compound of the formula (I) according to claim 1 and a high molecular

weight organic material.

**Revendications**

1. Procédé de coloration dans la masse de matière organique de haut poids moléculaire caractérisé par l'utilisation d'un composé de formule (I)

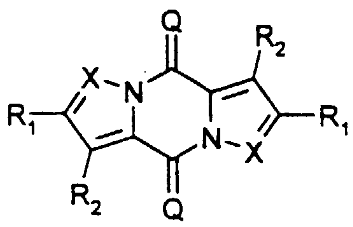

(I)

où

| | |
|---|---|
| Q | représente des atomes de O ou de S, et |
| X | représente un groupe $C(R_3)$ ou un atome de N, |
| $R_1$, $R_2$ et $R_3$ | représentent, indépendamment les uns des autres, des atomes d'hydrogène, des atomes d'halogènes pris dans le groupe comprenant des atomes de chlore, de brome et de fluor, des groupes alkyle en $C_1$-$C_6$, alkoxy en $C_1$-$C_6$, hydroxy, cyano, nitro, amino, morpholino, NH-alkyle en $C_1$-$C_6$ |

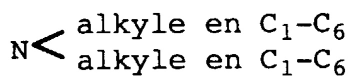

,

haloalkyle en $C_1$-$C_6$, NH-$E_1$, $A_1$ ou

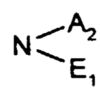

,

où

| | |
|---|---|
| $A_1$ et $A_2$ | représentent, indépendamment l'un de l'autre, un groupe aryle en $C_6$-$C_{12}$ substitué par 3 restes $R_4$, $R_5$ et $R_6$, ou un groupe hétéroaryle en $C_3$-$C_{12}$ qui contient dans le système cyclique 1 à 3 hétéroatomes pris dans le groupe comprenant des atomes de N, de O et de S, substitué par 3 restes $R_4$, $R_5$ et $R_6$, et |
| $E_1$ | représente des groupes alkyle en $C_1$-$C_6$, |

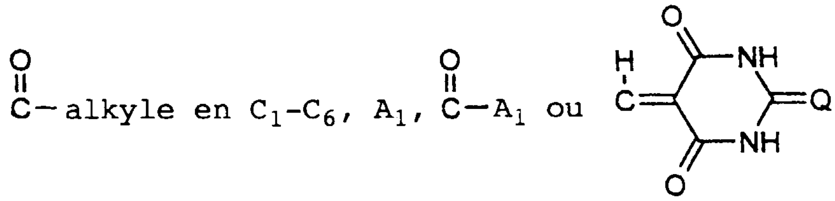

,

ou

$R_1$ et $R_2$ ensemble ou $R_1$ et $R_3$ ensemble représentent un reste $A_3$, où $A_3$ représente un groupe aralcénylène

en $C_8$-$C_{12}$ ou alcénylène en $C_4$-$C_8$ substitué par 3 restes $R_7$, $R_8$ et $R_9$,

et

$R_4$ à $R_9$ représentent, indépendamment les uns des autres, des atomes d'hydrogène, des atomes d'halogènes pris dans le groupe comportant des atomes de chlore, de brome et de fluor, des groupes alkyle en $C_1$-$C_6$, alkoxy en $C_1$-$C_6$, hydroxy, cyano, nitro, amino, morpholino, NH-alkyle en $C_1$-$C_6$,

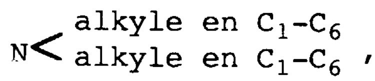

haloalkyle en $C_1$-$C_6$,

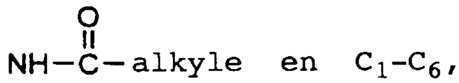

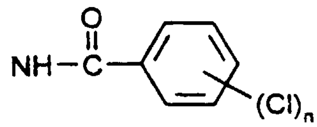

ou

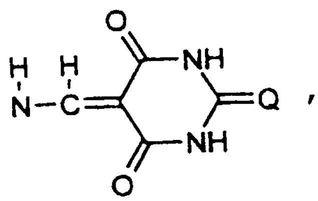

où n vaut zéro ou un nombre entier de 1 à 3.

**2.** Procédé selon la revendication 1, où dans la formule (I), Q représente un atome de O.

**3.** Procédé selon la revendication 1, où X représente un atome de N, et $R_1$ et $R_2$ représentent des groupes alkyle en $C_1$-$C_4$, alkoxy en $C_1$-$C_4$, amino, $A_1$ ou NH-$E_1$.

**4.** Procédé selon la revendication 3, où $A_1$ représente un groupe aryle en $C_6$-$C_{12}$ substitué par 3 restes $R_{10}$, $R_{11}$ et $R_{12}$, où $R_{10}$ à $R_{12}$ représentent, indépendamment les uns des autres, $R_4$ à $R_6$ selon la revendication 1.

**5.** Procédé selon la revendication 4, où $R_{10}$ à $R_{12}$ représentent des atomes d'hydrogène, des groupes alkoxy en $C_1$-$C_6$, amino,

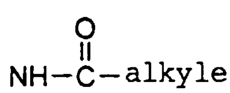

en $C_1$-$C_6$,

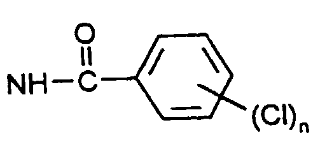

ou

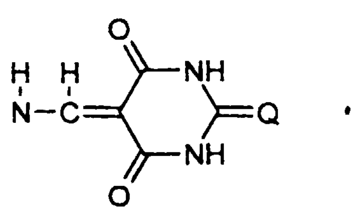

**6.** Procédé selon la revendication 1, où X représente $C(R_3)$, un reste $R_1$ ou $R_2$ représente des groupes alkyle en $C_1$-$C_4$, alkoxy en $C_1$-$C_4$, amino, $A_1$ ou NH-$E_1$, et l'autre reste $R_2$ ou $R_1$ conjointement avec $R_3$ forme un reste

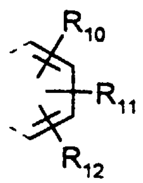, 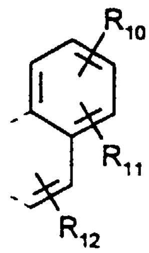 ou 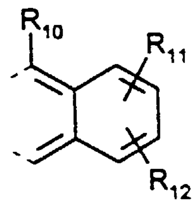,

$R_{10}$ à $R_{12}$ possédant la définition donnée à la revendication 4.

**7.** Procédé selon la revendication 6, où $R_1$ conjointement avec $R_3$ représente un reste

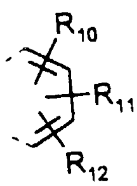, 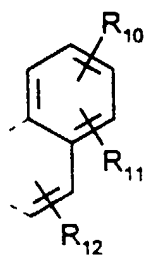 ou 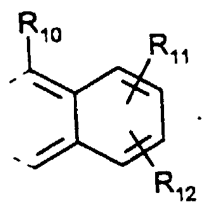.

**8.** Procédé selon la revendication 1, où

1] X représente un atome de N, $R_1$ représente des groupes méthyle, éthyle ou $A_1$, et $R_2$ représente des groupes amino ou NH-$E_1$ ;
2] X représente un atome de N, $R_1$ représente un reste $A_1$, et $R_2$ représente un atome d'hydrogène ;
3] X représente un atome de N, $R_1$ représente un atome d'hydrogène, et $R_2$ représente des groupes amino, morpholino,

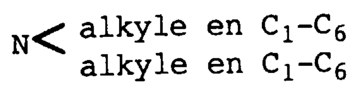,

NH-$E_1$ ou

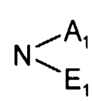

4] X représente un groupe $C(R_{13})$, $R_1$ et $R_2$ ensemble représentent un reste

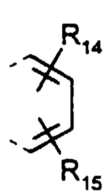,

$R_{13}$ représente des groupes amino ou NH-$E_1$, et $R_{14}$ ainsi que $R_{15}$ représentent, indépendamment l'un de l'autre, des atomes d'hydrogène, des groupes amino, NH-$E_1$ ou nitro.

**9.** Procédé selon la revendication 1, où le composé de formule (I) présente, au moins dans une forme tautomère, au moins deux atomes d'hydrogène liés chacun à un atome d'azote.

**10.** Procédé selon la revendication 1, où le composé de formule (I) présente une surface spécifique selon BET de 5 à 150 $m^2$/g.

**11.** Procédé selon la revendication 1, où on utilise le composé de formule (I) en une quantité de 0,1 à 30 % massiques, de préférence de 0,1 à 10 % massiques par rapport à la matière organique de haut poids moléculaire à pigmenter.

**12.** Composé de formule

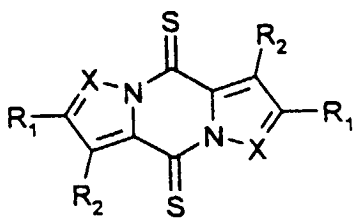 (IV),

où X, $R_1$ et $R_2$ possèdent la même définition qu'à la formule (I) selon la revendication 1.

**13.** Composé de formule

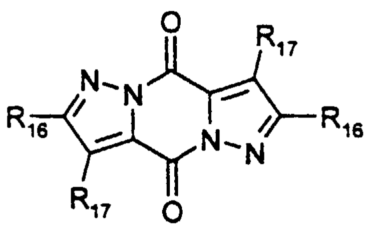 (V),

où

- $R_{16}$ représente un groupe aryle en $C_6$-$C_{12}$, lequel est substitué par au moins un reste alkoxy en $C_1$-$C_6$, amino, morpholino, NH-alkyle en $C_1$-$C_6$,

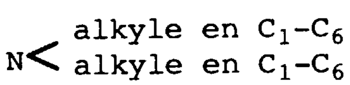 , NH-$E_2$ ou 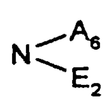 ,

et

$R_{17}$ représente indépendamment de $R_{16}$ des atomes d'hydrogène, de chlore, de brome, de fluor, des groupes alkyle en $C_1$-$C_6$, alkoxy en $C_1$-$C_6$, hydroxy, cyano, nitro, amino, morpholino

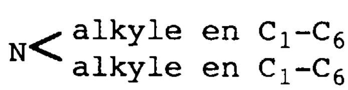 ,

haloalkyle en $C_1$-$C_6$, NH-$E_2$, $A_5$ ou

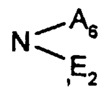

- à condition que $R_{17}$ ne représente pas un atome d'hydrogène, lorsque $R_{16}$ représente un groupe

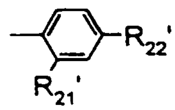

où $R'_{21}$ représente un groupe alkoxy et $R'_{22}$ représente un atome d'hydrogène, des groupes alkyle, alkoxy, halogène ou hydroxy, ou $R'_{22}$ représente un groupe alkoxy et $R'_{21}$ représente un atome d'hydrogène, des groupes alkyle, alkoxy, halogène ou hydroxy ;

ou

♦ $R_{16}$ représente des atomes d'hydrogène, de chlore, de brome, de fluor, des groupes alkyle en $C_1$-$C_6$, alkoxy $C_1$-$C_6$, hydroxy, cyano, nitro, amino, morpholino,

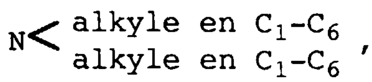

haloalkyle en $C_1$-$C_6$, NH-$E_2$, $A_5$ ou

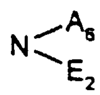

et

$R_{17}$ représente indépendamment de $R_{16}$ des groupes alkoxy en $C_1$-$C_6$, amino, morpholino,

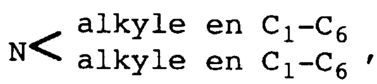 , NH-$E_2$, 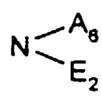

ou $A_5$ ;
ou

♦ $R_{16}$ et $R_{17}$ représente ensemble un reste $A_7$, où

$E_2$ représente des groupes alkyle en $C_1$-$C_6$,

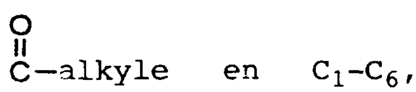 $A_5$, 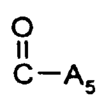

ou

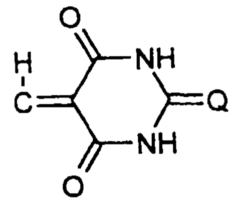

A$_5$ et A$_6$ représentent, indépendamment l'un de l'autre, un groupe aryle en $C_6$-$C_{12}$ substitué par 3 restes $R_{21}$, $R_{22}$ et $R_{23}$ ou un groupe hétéroaryle en $C_3$-$C_{12}$ qui contient dans le système cyclique 1 à 3 hétéroatomes pris parmi les atomes N, O et S substitué par 3 restes $R_{21}$, $R_{22}$ et $R_{23}$,

$A_7$ représente alcénylène en $C_4$-$C_8$ ou aralcénylène en $C_8$-$C_{12}$ substitué par 3 restes $R_{24}$, $R_{25}$ et $R_{27}$,

$R_{21}$ à $R_{25}$ représentent, indépendamment les uns des autres, des atomes d'hydrogène, de chlore, de brome, de fluor, des groupes alkyle en $C_1$-$C_6$, alkoxy en $C_1$-$C_6$, hydroxy, cyano, nitro, amino, morpholino, NH-alkyle en $C_1$-$C_6$,

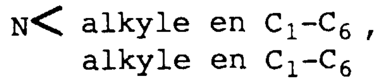

haloalkyle en $C_1$-$C_6$,

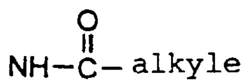

en $C_1$-$C_6$,

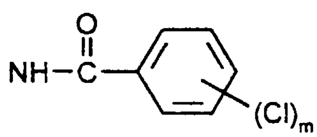

ou

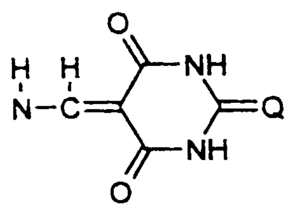

,

où m vaut zéro ou un nombre entier de 1 à 3 ;

$R_{27}$ représente un groupe alkoxy en $C_1$-$C_6$, et

Q représente un atome de O ou de S.

**14.** Composé de formule

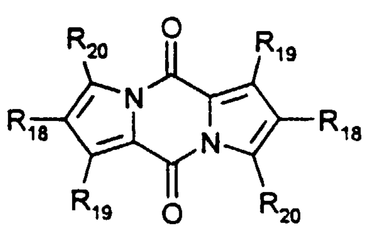

(VI),

où

- $R_{18}$ représente des atomes d'hydrogène, de chlore, de brome, de fluor, des groupes alkyle en $C_1$-$C_6$, alkoxy

en $C_1$-$C_6$, hydroxy, cyano, nitro, amino, morpholino,

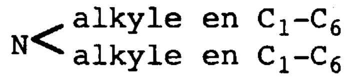 ,

haloalkyle en $C_1$-$C_6$, NH-$E_3$, $A_8$ ou

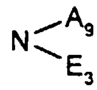 ,

$R_{19}$ représente des groupes alkoxy en $C_1$-$C_6$, amino, morpholino, NH-alkyle en $C_1$-$C_6$,

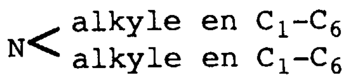 , NH-$E_3$, $A_8$ ou 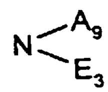 ,

et

$R_{20}$ représente des atomes d'hydrogène, de chlore, de brome, de fluor, des groupes alkyle en $C_1$-$C_6$, alkoxy en $C_1$-$C_6$, hydroxy, cyano, nitro, amino, morpholino,

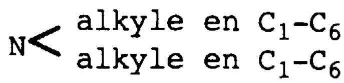 ,

haloalkyle en $C_1$-$C_6$, NH-$E_3$ ou

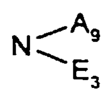 ;

ou

- ♦ $R_{18}$ représente des groupes alkoxy en $C_1$-$C_6$, amino, morpholino, NH-alkyle en $C_1$-$C_6$,

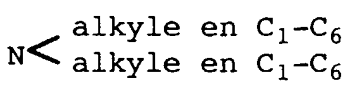 , NH-$E_3$ ou 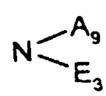 ,

$R_{19}$ représente des atomes d'hydrogène, de chlore, de brome, de fluor, des groupes alkyle en $C_1$-$C_6$, alkoxy, en $C_1$-$C_6$, hydroxy, cyano, nitro, amino, morpholino,

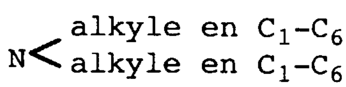 ,

haloalkyle en $C_1$-$C_6$, NH-$E_3$, $A_8$ ou

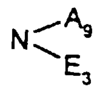 ,

et

$R_{20}$ représente un reste $A_8$ ;

ou

- $R_{18}$ et $R_{19}$ représentent, indépendamment l'un de l'autre, des atomes d'hydrogène, de chlore, de brome, de fluor, des groupes alkyle en $C_1$-$C_6$, alkoxy en $C_1$-$C_6$, hydroxy, cyano, nitro, amino, morpholino,

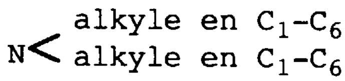 ,

haloalkyle en $C_1$-$C_6$, NH-$E_3$, $A_8$ ou

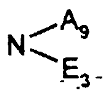 ,

et

$R_{20}$ représente un reste $A_{12}$ ;

ou

- $R_{18}$ et $R_{19}$ ensemble représentent un reste $A_{11}$, et $R_{20}$ représente un atome d'hydrogène, des atomes d'halogènes, de chlore, de brome, de fluor, des groupes alkyle en $C_1$-$C_6$, alkoxy en $C_1$-$C_6$, hydroxy, cyano, nitro, amino, morpholino,

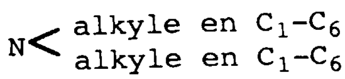 ,

haloalkyle en $C_1$-$C_6$, NH-$E_3$, $A_8$ ou

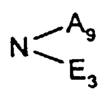 ,

ou

- $R_{18}$ et $R_{20}$ ensemble représentent un reste $A_{11}$, et $R_{19}$ représente, des groupes alkyle en $C_1$-$C_6$ ou amino,

ou

- $R_{18}$ et $R_{20}$ ensemble représentent un reste $A_{10}$, et $R_{19}$ représente des atomes de chlore, de brome, de fluor, des groupes alkoxy en $C_1$-$C_6$, hydroxy, cyano, nitro, morpholino,

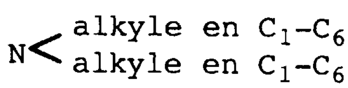 ,

haloalkyle en $C_1$-$C_6$, NH-$E_3$, $A_8$ ou

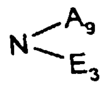 ,

- à condition qu'il ne s'agisse pas pour le composé de formule (VI) d'un composé de formule

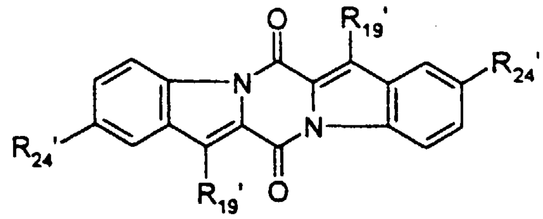 (XXIV),

où lorsque $R'_{24}$ représente un atome d'hydrogène, $R'_{19}$ représente des groupes cyano ou hydroxy, ou lorsque $R'_{24}$ représente un atome de chlore, $R'_{19}$ représente groupes phényle, o-chloro-phényle ou o-fluoro-phényle ;

où

$E_3$ représente des groupes alkyle en $C_1$-$C_6$,

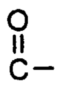 alkyle en $C_1$-$C_6$, $A_8$, 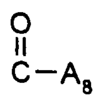

ou

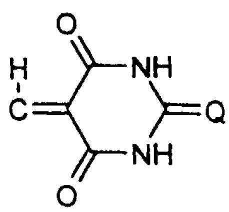 ;

$A_8$ et $A_9$ représentent, indépendamment l'un de l'autre, un groupe aryle en $C_6$-$C_{12}$ substitué par 3 restes $R_{21}$, $R_{22}$ et $R_{23}$, ou un groupe hétéroaryle en $C_3$-$C_{12}$ qui contient dans le système cyclique 1 à 3 hétéroatomes pris dans le groupes comprenant des atomes de N, de O et de S, substitué par 3 restes $R_{21}$, $R_{22}$ et $R_{23}$,

$A_{10}$ représente un groupe alcénylène en $C_4$-$C_8$ ou arylène en $C_8$-$C_{12}$ substitué par 3 restes $R_{24}$, $R_{25}$ et $R_{26}$,

$A_{11}$ représente un groupe alcénylène en $C_4$-$C_8$ ou arylène en $C_8$-$C_{12}$ substitué par 3 restes $R_{24}$, $R_{25}$ et $R_{27}$,

$A_{12}$ représente un groupe aryle en $C_6$-$C_{12}$ substitué par 3 restes $R_{21}$, $R_{22}$ et $R_{28}$, ou un groupe hétéroaryle en $C_3$-$C_{12}$ qui contient dans le système cyclique 1 à 3 hétéroatomes pris dans le groupe comportant des atomes de N, de O et de S, substitué par 3 restes $R_{21}$, $R_{22}$ et $R_{28}$ ;

$R_{21}$ à $R_{26}$ représentent, indépendamment les uns des autres, des atomes d'hydrogène, de chlore, de brome, de fluor, des groupes alkyle en $C_1$-$C_6$, alkoxy en $C_1$-$C_6$, hydroxy, cyano, nitro, amino, morpholino, NH-alkyle en $C_1$-$C_6$,

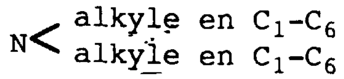

haloalkyle en $C_1$-$C_6$,

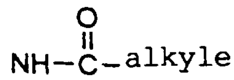

en $C_1$-$C_6$,

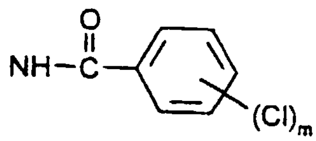 ou 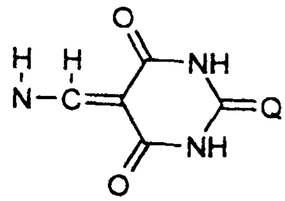 ;

$R_{27}$ et $R_{28}$ représentent, indépendamment l'un de l'autre, des groupes alkoxy en $C_1$-$C_6$, amino, morpholino, NH-alkyle en $C_1$-$C_6$,

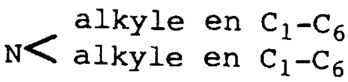 , $NH-E_3$ ou 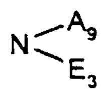 ;

m vaut zéro ou un entier de 1 à 3 ; et
Q représente un atome de O ou de S.

**15.** Composé selon la revendication 12, 13 ou 14 qui présente au moins un groupe alkoxy en $C_1$-$C_6$, amino, morpholino,

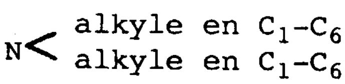 , $NH-E_2$ ou 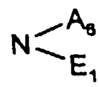 ,

où $E_1$, $E_2$ et $A_6$ possèdent la définition donnée à la revendication 12.

**16.** Composition de substances contenant un composé de formule (I) selon la revendication 1 et une matière organique de haut poids moléculaire.